(19) Europäisches Patentamt
European Patent Office
Office européen des brevets

(11) **EP 3 856 038 B1**

(12) **EUROPÄISCHE PATENTSCHRIFT**

(45) Veröffentlichungstag und Bekanntmachung des Hinweises auf die Patenterteilung:
**13.03.2024   Patentblatt 2024/11**

(21) Anmeldenummer: **19778938.1**

(22) Anmeldetag: **24.09.2019**

(51) Internationale Patentklassifikation (IPC):
*A61B 8/06* (2006.01)    *A61B 8/08* (2006.01)
*A61B 8/12* (2006.01)    *A61M 60/178* (2021.01)
*A61M 60/216* (2021.01)    *A61M 60/523* (2021.01)

(52) Gemeinsame Patentklassifikation (CPC):
**A61B 8/488; A61B 8/065; A61B 8/0883;**
**A61B 8/12; A61M 60/178; A61M 60/216;**
**A61M 60/523; A61M 60/816;** A61M 60/148

(86) Internationale Anmeldenummer:
**PCT/EP2019/075662**

(87) Internationale Veröffentlichungsnummer:
**WO 2020/064707 (02.04.2020 Gazette 2020/14)**

(54) **VERFAHREN UND SYSTEM ZUR BESTIMMUNG EINER STRÖMUNGSGESCHWINDIGKEIT EINES DURCH EIN IMPLANTIERTES, VASKULÄRES UNTERSTÜTZUNGSSYSTEM STRÖMENDEN FLUIDS**

METHOD AND APPARATUS FOR DETERMINING VELOCITY OF A FLUID FLOWING THROUGH A VASCULAR ASSSIT SYSTEM

PROCÉDÉ ET DISPOSITF POUR LA DÉTERMINATION DE LA VITESSE D'UN FLUIDE CIRCULANT DANS UN SYSTÈME DE SOUTIEN VASCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.09.2018   DE 102018216305**

(43) Veröffentlichungstag der Anmeldung:
**04.08.2021   Patentblatt 2021/31**

(73) Patentinhaber: **Kardion GmbH**
**70376 Stuttgart (DE)**

(72) Erfinder:
• SCHLEBUSCH, Thomas, Alexander
 71272 Renningen (DE)
• SCHMID, Tobias
 70197 Stuttgart (DE)

(74) Vertreter: **Gauss, Nikolai et al**
**Pfiz/Gauss Patentanwälte PartmbB**
**Tübingerstraße 26**
**70178 Stuttgart (DE)**

(56) Entgegenhaltungen:
DE-A1- 19 520 920      US-A1- 2008 133 006
US-A1- 2016 000 983

**Beschreibung**

Die Erfindung betrifft ein Verfahren und ein System zur Bestimmung eines Strömungsparameters von durch ein implantiertes, vaskuläres Unterstützungssystem strömendem Blut. Die Erfindung betrifft auch ein implantierbares, vaskuläres Unterstützungssystem zur Durchführung des Verfahrens. Die Erfindung findet insbesondere Anwendung bei (voll-)implantierten Linksherz-Unterstützungssystemen (LVAD).

Es ist bekannt Ultraschall-Volumenstromsensoren in Herzunterstützungssysteme zu integrieren, um mit ihnen den sogenannten Pumpenvolumenstrom zu erfassen, der den Fluid-Volumenstrom durch das Unterstützungssystem selbst quantifiziert. Die Ultraschall-Volumenstromsensoren können dabei gepulste Dopplermessungen durchführen bzw. das gepulste Doppler-(engl.: Pulsed Wave Doppler; kurz: PWD)-Verfahren einsetzen. Dieses benötigt nur ein Ultraschall-Wandlerelement und ermöglicht eine genaue Wahl des Abstandes des Beobachtungsfensters vom Ultraschall-Element. Bei bekannten PWD-Systemen werden Ultraschallimpulse mit einer festgelegten Pulswiederholrate (PRF) ausgesendet. Dabei muss die Pulswiederholrate die doppelte, maximal auftretende Doppler-Frequenzverschiebung übertreffen, um das Nyquist-Theorem nicht zu verletzten. Ist diese Bedingung nicht erfüllt, kommt es zu Aliasing, d. h. Mehrdeutigkeiten im erfassten Frequenzspektrum.

Aufgrund der geometrischen Gestaltung des Messaufbaus in Herzunterstützungssystemen (VAD) liegt der Messbereich bzw. das Beobachtungsfenster unter Umständen so weit vom Ultraschallwandler entfernt, dass die Signallaufzeit des Ultraschallimpulses vom Wandler zum Messbereich und zurück zum Wandler nicht vernachlässigt werden kann. Da beim PWD-Verfahren ein neuer Ultraschallimpuls (zumindest theoretisch) erst ausgesendet werden darf bzw. sollte, wenn der vorangegangene keine signifikanten Echos mehr liefert, limitiert die Signallaufzeit die maximal mögliche Pulswiederholrate. Bei den üblicherweise hohen, in Herzunterstützungssystemen vorherrschenden Strömungsgeschwindigkeiten und den geometrischen Randbedingungen für die Entfernung des Beobachtungsfensters vom Ultraschallelement kommt es in der Regel zu einer Verletzung des Nyquist-Abtasttheorems, wodurch Mehrdeutigkeiten (Aliasing) im Spektrum entstehen.

Herzunterstützungssysteme mit Ultraschallsensoren, die nicht das PWD-Verfahren anwenden, sind üblicherweise mit zwei Ultraschallwandlern ausgestattet, sodass die beschriebene Laufzeitproblematik zwar auftreten kann, aber bei entsprechender Umsetzung anderweitig gelöst werden kann. Herzunterstützungssysteme mit Ultraschallsensoren, die das PWD-Verfahren anwenden sind jedoch insbesondere für mittlere bis hohe Flussgeschwindigkeiten anfällig für den beschriebenen Effekt. Stand der Technik ist derzeit die Maßgabe, die festgelegte Pulswiederholrate so zu wählen, dass Aliasing nicht auftritt.

Die US 2008/133006 A1 betrifft eine implantierbare Blutpumpe mit einem Ultraschallwandler zum Erfassen und Messen des Blutflusses und zum Ermöglichen einer Ultraschallbildgebung, wobei der Ultraschallwandler innerhalb der Blutpumpe angebracht ist. Die US 2016/0000983 A1 beschreibt ein Herzunterstützungssystem, das einen Ultraschall-Dopplersensor enthält, um damit die Strömungsgeschwindigkeit von Blut an dessen Auslass zu bestimmen. Dieser Dopplersensor dient somit nicht für das Messen der Strömungsgeschwindigkeit von Blut in einem kanülenartigen Abschnitt.

In der DE 195 20 920 A1 ist ein Ultraschall-Dopplersensor zum Bestimmen des Geschwindigkeit-Zeit-Spektrums auf der Grundlage einer gepulsten Dopplermessung einer Blutströmung angegeben. Hier gibt es jedoch keine Angaben dazu, ob und wie dieser Dopplersensor und dessen Messfenster in einem Herzunterstützungssystem angeordnet ist.

Aufgabe der Erfindung ist es, ein Verfahren anzugeben und ein System bereitzustellen, mittels dessen mindestens ein Strömungsparameter von durch ein implantiertes, vaskuläres Unterstützungssystem strömendem Blut, insbesondere ein Strömungsparameter von durch ein implantiertes, vaskuläres Unterstützungssystem strömendem Blut zuverlässig und genau bestimmt werden kann.

Diese Aufgabe wird durch das in Anspruch 1 angegebene Verfahren und das in Anspruch 8 angegebene System gelöst. Vorteilhafte Ausführungsformen der Erfindung sind in den abhängigen Ansprüchen angegeben.

Hier vorgeschlagen wird gemäß Anspruch 1 ein Verfahren zur Bestimmung mindestens eines Strömungsparameters, d.h. insbesondere einer Strömungsgeschwindigkeit von durch einen kanülenartigen Abschnitt eines implantierten, vaskulären Unterstützungssystems strömendem Blut, umfassend folgende Schritte:

a) Schätzen der Strömungsgeschwindigkeit des Bluts,

b) Durchführen einer gepulsten Dopplermessung mittels eines Ultraschallsensors des Unterstützungssystems in einem Beobachtungsfenster innerhalb des kanülenartigen Abschnitts des Unterstützungssystems, wobei ein Verschieben des Beobachtungsfensters mit einer Beobachtungsfenstergeschwindigkeit erfolgt, welche unter Verwendung der in Schritt a) geschätzten Strömungsgeschwindigkeit bestimmt wird,

c) Ermitteln des mindestens einen Strömungsparameters des Bluts unter Verwendung zumindest eines Messergebnisses der gepulsten Dopplermessung oder eines Messergebnisses der gepulsten Dopplermessung und der Beobachtungsfenstergeschwindigkeit.

Das vaskuläre Unterstützungssystem ist bevorzugt ein kardiales Unterstützungssystem (bzw. Herzunterstützungssystem), besonders bevorzugt ein ventrikuläres Unterstützungssystem. Regelmäßig dient das Unterstützungssystem zur Unterstützung der Förderung von Blut im Blutkreislauf eines Menschen, ggf. Patienten. Das Unterstützungssystem kann zumindest teilweise in einem Blutgefäß angeordnet sein. Bei dem Blutgefäß handelt es sich beispielsweise um die Aorta, insbesondere bei einem Linksherz-Unterstützungssystem, oder um den gemeinsamen Stamm (Truncus pulmonalis) in die beiden Lungenarterien, insbesondere bei einem Rechtsherz-Unterstützungssystem. Das Unterstützungssystem ist bevorzugt am Ausgang des linken Ventrikels des Herzens bzw. der linken Herzkammer angeordnet. Besonders bevorzugt ist das Unterstützungssystem in Aortenklappenposition angeordnet.

Das Verfahren kann zur Bestimmung einer Fluid-Strömungsgeschwindigkeit und/oder eines Fluid-Volumenstroms aus einem Ventrikel eines Herzens, insbesondere von einem (linken) Ventrikel eines Herzens hin zur Aorta im Bereich eines (voll-)implantierten, (links-)ventrikulären (Herz-)Unterstützungssystems beitragen. Bei dem Fluid handelt es sich regelmäßig um Blut. Bei der Strömungsgeschwindigkeit handelt es sich in der Regel um eine Hauptgeschwindigkeitskomponente einer Fluidströmung, insbesondere Blutströmung. Die Strömungsgeschwindigkeit wird in einem Fluidstrom bzw. Fluid-Volumenstrom bestimmt, der durch das Unterstützungssystem, insbesondere durch einen Kanal des Unterstützungssystems hindurch strömt. Das Verfahren ermöglicht in vorteilhafter Weise, dass die Strömungsgeschwindigkeit und/oder der Fluid-Volumenstrom des Blut-Flusses auch außerhalb des OP-Szenarios mit hoher Qualität bestimmt werden kann, insbesondere durch das implantierte Unterstützungssystem selbst.

Die hier vorgeschlagene Lösung trägt insbesondere zur Kompensation von Aliasingeffekten (bzw. "spectrum wrapping") in einem medizinischen Pulsed Wave Doppler System bei. Darüber hinaus kann die hier vorgestellte Methode auch in vorteilhafter Weise zu einer Reduktion der spektralen Verbreiterung des Peaks im Dopplerspektrum beitragen, der die verschiedenen im Blutfluss vorkommenden und in den Frequenzbereich transformierten Blutflussgeschwindigkeiten darstellt. Insbesondere dient das Verfahren zur Elimination von Aliasingeffekten, Verringerung der spektralen Breite des Signals bewegter Streuer und/oder Verbreiterung des Signals statischer Streuer bei Messung eines durch ein implantiertes, vaskuläres Unterstützungssystem strömenden Fluids.

Ein zentraler Aspekt der hier vorgestellten Lösung kann insbesondere auch darin gesehen werden, ein Verschieben des Beobachtungsfensters des PWD-Messaufbaus mit der ungefähren Flussgeschwindigkeit des Bluts durchzuführen, sodass die entstehende Differenzgeschwindigkeit zwischen Blut und der Verschiebegeschwindigkeit des Beobachtungsfensters in einen mit der gewählten Pulswiederholrate (PRF) eindeutig messbaren Bereich abgebildet werden kann. Die sich aus dieser Differenzgeschwindigkeit ergebende Dopplerfrequenz ist in vorteilhafter Weise mehrdeutigkeitsfrei bestimmbar.

Dies trägt insbesondere dazu bei, dass folgende besonders vorteilhaften Effekte (zumindest in einem signifikanten Maße) erzielt werden können:

- Aliasing wird eliminiert, insbesondere indem eine Eindeutigkeit der Dopplerfrequenz hergestellt wird.
- Durch bewegte Streuer hervorgerufene Frequenzpeaks im Dopplerspektrum werden verschmälert. Damit steigt ihre Amplitude und sie treten aus dem Hintergrundrauschen (besser) hervor. Außerdem wird das Auflösungsvermögen des Messsystems erhöht.
- Durch statische Streuer hervorgerufene Frequenzpeaks im Dopplerspektrum werden verbreitert. Damit sinkt ihre Amplitude ab und die Signalenergie wird im Spektrum verschmiert.

Ein insbesondere neben der Elimination von Aliasingeffekten hervorzuhebender Effekt ist in diesem Zusammenhang, dass Dopplersignale bewegter Streuer verschmälert werden. Dies kann mit anderen Worten auch als eine Verringerung der "Spektralen Verbreiterung" (der Hauptfrequenzkomponente) der gemessenen relativen Dopplerverschiebung zwischen Beobachtungsfenstergeschwindigkeit und Strömungsgeschwindigkeit beschrieben werden. Insbesondere wird durch eine Verschiebung eines Beobachtungsfensters in Strömungsrichtung die Verweildauer der Streuer im Beobachtungsfenster erhöht und dadurch die spektrale Breite der Dopplerfrequenzanteile der entsprechenden Strömungsgeschwindigkeiten(en) im Spektrum reduziert.

Insbesondere wirkt derselbe Effekt umgekehrt auf die statischen Streuer z. B. im umgebenden Gewebe. Die kürzere Verweildauer im Beobachtungsfenster führt zu einer Verschmierung der Energie im Spektrum, was das Störpotential dieser Reflexionen reduziert.

In Schritt a) erfolgt ein Schätzen der Strömungsgeschwindigkeit des Bluts. Dies bedeutet mit anderen Worten insbesondere, dass in Schritt a) die Strömungsgeschwindigkeit, welche in Schritt c) ermittelt (z. B. berechnet) werden soll, vorab (grob) geschätzt wird. Vorteilhafterweise fußt diese Schätzung auf einer zuvor durchgeführten Ultraschallmessung (z. B. mit festem Beobachtungsfenster) mittels des Ultraschallsensors des Unterstützungssystems. Dies ist jedoch nur beispielhaft. So könnte die Schätzung z. B auch auf einem Erfahrungswert, beispielsweise basierend auf dem Alter und/oder ggf. der Schwere der Erkrankung des Patienten beruhen. Entsprechende Erfahrungswerte könnten beispielsweise in einer Tabelle hinterlegt sein, auf die ein Steuergerät des Unterstützungssystems zugreifen kann. Weiterhin kann vorgesehen sein, dass die Strömungsgeschwindigkeit, insbesondere bei hohen Unterstützungsgraden auf Basis der elektronischen Leistung einer Strömungsmaschine des Unterstützungssystems geschätzt wird.

In Schritt b) erfolgt ein Durchführen einer gepulsten Dopplermessung mittels eines Ultraschallsensors des Unterstützungssystems in einem Beobachtungsfenster innerhalb des Unterstützungssystems, wobei ein Verschieben des Beobachtungsfensters mit einer Beobachtungsfenstergeschwindigkeit erfolgt, welche unter Verwendung der in Schritt a) geschätzten Strömungsgeschwindigkeit bestimmt wird. Bei dem Ultraschallsensor kann es sich beispielsweise um einen Ultraschallwandler handeln. Bevorzugt ist ein einzelner (nur ein) Ultraschallsensor vorgesehen. Der Ultraschallsensor umfasst weiterhin bevorzugt ein einzelnes (nur ein) Ultraschall-Element, das vorteilhafter Weise mit einem Piezo-Element gebildet ist.

Das Beobachtungsfenster befindet sich (stets) in einem (insbesondere möglichst gleichmäßig durchströmten) Kanal des Unterstützungssystems. In der Regel durchläuft eine Hauptabstrahlrichtung des Ultraschallsensors den Kanal des Unterstützungssystems und das Beobachtungsfenster (mittig bzw. zentral). Insbesondere zeigt die Hauptabstrahlrichtung des Ultraschallsensors in den Kanal des Unterstützungssystems. Vorzugsweise durchläuft das Beobachtungsfenster diesen Bereich (den Kanal) möglichst radial mittig ausgerichtet. Das Verschieben des Beobachtungsfensters mit der Beobachtungsfenstergeschwindigkeit erfolgt regelmäßig entlang der Hauptabstrahlrichtung des Ultraschallsensors und entlang der Strömungsrichtung des Fluids. Die Beobachtungsfenstergeschwindigkeit wird insbesondere in Abhängigkeit der in Schritt a) geschätzten Strömungsgeschwindigkeit bestimmt. Die Verschiebung des Beobachtungsfensters erfolgt insbesondere mit der zuvor bestimmten (geschätzten), ungefähren Flussgeschwindigkeit des Fluids (Blutes).

In Schritt c) erfolgt ein Ermitteln des mindestens einen Strömungsparameters des Fluids unter Verwendung eines Messergebnisses der gepulsten Dopplermessung und/oder eines Messergebnisses der gepulsten Dopplermessung und der Beobachtungsfenstergeschwindigkeit. Vorzugsweise handelt es sich bei dem Strömungsparameter um eine Strömungsgeschwindigkeit und/oder einen Fluidvolumenstrom. Bei bekanntem durchströmbaren Querschnitt (beispielsweise des Kanals) und bekanntem Strömungsprofil kann der Fluidvolumenstrom unmittelbar aus der Strömungsgeschwindigkeit ermittelt werden.

Hierzu werden gegebenenfalls hinterlegte Kalibrierdaten verwendet. Diese Kalibrierdaten ermöglichen es von einer Zentralflussgeschwindigkeit unter Annahme des für jede Zentralflussgeschwindigkeit geltenden Strömungsprofils auf die mittlere Flussgeschwindigkeit zu schließen und dann durch Multiplikation mit dem Querschnitt den Volumenfluss zu berechnen. Gegebenenfalls kann der Beitrag des Strömungsprofils auch durch eine abschließende Korrektur des Volumenstroms berechnet werden.

Alternativ oder kumulativ zu der Strömungsgeschwindigkeit und/oder dem Fluidvolumenstrom kann es sich bei dem Strömungsparameter auch beispielsweise um eine (Blut-)Viskosität und/oder einen Haematokritwert handeln. Dies soll insbesondere verdeutlichen, dass das (nach der hier vorgestellten Lösung verbesserte) Dopplerspektrum auch abseits der Strömungsgeschwindigkeitsmessung weiterverarbeitet bzw. genutzt werden kann. Beispielsweise kann das (nach der hier vorgestellten Lösung verbesserte) Dopplerspektrum als ein Eingangsdatensatz für weitere Signalverarbeitungsverfahren zur Bestimmung weiterer Vital- und/oder Systemparameter verwendet werden, beispielsweise zur Abschätzung der Blutviskosität und/oder des Haematokritwerts.

Das Messergebnis der gepulsten Dopplermessung kann beispielsweise in Form eines Peaks vorliegen. Beispielsweise im Wege einer Kalibrierung können bestimmten Peaks im Spektrum bestimmte Werte des Strömungsparameters, etwa bestimmte Werte für die Geschwindigkeit, die Viskosität und/oder den Haematokritwert zugeordnet werden. Dies erlaubt ein beispielhaftes und besonders vorteilhaftes Ermitteln der genannten Parameter durch einen Vergleich mit hinterlegten Kalibrierdaten.

Nach einer vorteilhaften Ausgestaltung wird vorgeschlagen, dass in Schritt c) ein Ermitteln einer Strömungsgeschwindigkeit des Bluts unter Verwendung eines Messergebnisses der gepulsten Dopplermessung und der Beobachtungsfenstergeschwindigkeit erfolgt. Vorteilhafterweise erfolgt in Schritt c) eine Berechnung des Dopplerspektrums der Differenzgeschwindigkeit zwischen der Strömungsgeschwindigkeit des Bluts (Blutflussgeschwindigkeit) und der Beobachtungsfenstergeschwindigkeit (Verschiebegeschwindigkeit des Beobachtungsfensters). Weiterhin bevorzugt erfolgt in Schritt c) eine Ermittlung der Hauptfrequenzkomponente des Dopplerspektrums der Differenzgeschwindigkeit (z. B. einfacher Frequenzpeak oder template matching der erwarteten Frequenzverteilung).

Insbesondere wird in Schritt b) ein neuer Ultraschall-Impuls erst ausgesendet, wenn ein Echo (aus einem gewünschten Beobachtungsfensterabstand zum Ultraschallelement) eines unmittelbar zuvor ausgesendeten Ultraschall-Impulses empfangen wurde. Bevorzugt wird ein neuer Ultraschall-Impuls erst ausgesendet, wenn alle (signifikanten) Echos eines unmittelbar zuvor ausgesendeten Ultraschall-Impulses empfangen wurden.

Nach einer vorteilhaften Ausgestaltung wird vorgeschlagen, dass in Schritt a) die Schätzung auf Basis mindestens eines Betriebsparameters einer Strömungsmaschine des Unterstützungssystems erfolgt. Dies bedeutet mit anderen Worten insbesondere, dass die in Schritt a) die Strömungsgeschwindigkeit vorteilhafterweise auf Basis bzw. in Abhängigkeit mindestens eines Betriebsparameters einer Strömungsmaschine des Unterstützungssystems geschätzt wird. Dabei kann in besonders vorteilhafter Weise der Sachverhalt genutzt werden, dass aufgrund des Motorkennfeldes des Strömungsmaschine eine grobe Abschätzung des Pumpenflusses (nur) aus der Drehrate des Antriebs oder auf Basis des Differenzdrucks über die Strömungsmaschine und der Drehrate möglich ist.

Vorzugsweist ist der Betriebsparameter der Strömungsmaschine zumindest eine Drehzahl, ein Strom, eine Leistung oder ein Druck. Bevorzugt ist der Betriebsparameter eine Drehzahl (bzw. Drehrate) der Strömungsmaschine, etwa eines Antriebs (z. B. eines Elektromotors) und/oder eines Schaufelrads der Strömungsmaschine. In der Regel ist die ungefähre Viskosität des Bluts des Patienten bekannt. Weiterhin bevorzugt umfasst der mindestens eine Betriebsparameter eine Drehzahl der Strömungsmaschine und einen Differenzdruck über die Strömungsmaschine. Bevorzugt wird mit dem Betriebsparameter eine geschätzte Strömungsgeschwindigkeit des Fluids ermittelt (geschätzt). Dies kann beispielsweise unter Verwendung eines Kennfeldes erfolgen, in dem die geschätzte Strömungsgeschwindigkeit in Abhängigkeit von dem mindestens einen Betriebsparamater hinterlegt ist

Alternativ oder kumulativ kann in Schritt a) eine Ultraschallmessung mittels des Ultraschallsensors des Unterstützungssystems durchgeführt werden. Bevorzugt handelt es sich bei dieser Ultraschallmessung nicht um eine gepulste Dopplermessung. Vielmehr kann hierbei z. B. eine Ultraschallmessung mit zwei Ultraschallsensoren durchgeführt werden, die hierzu beispielhaft in dem Unterstützungssystem vorgesehen sein könnten.

Nach einer vorteilhaften Ausgestaltung wird vorgeschlagen, dass die Beobachtungsfenstergeschwindigkeit so bestimmt wird, dass eine (relative) Dopplerverschiebung (zwischen Strömungsgeschwindigkeit und Beobachtungsfenstergeschwindigkeit) in einen Bereich transformiert wird, der mehrdeutigkeitsfrei darstellbar ist. Dies kann mit anderen Worten auch so beschrieben werden, dass das Beobachtungsfenster mit einer Geschwindigkeit bewegt wird, welche die relative Dopplerverschiebung zwischen Blutfluss und der Geschwindigkeit des Beobachtungsfensters in einen Bereich transformiert, der insbesondere mit der gewählten Ultraschallfrequenz und PRF mehrdeutigkeitsfrei darstellbar ist.

Vorzugsweise wird die Beobachtungsfenstergeschwindigkeit so bestimmt, dass zwischen der (geschätzten bzw. zu ermittelnden) Strömungsgeschwindigkeit des Fluids und der Beobachtungsfenstergeschwindigkeit eine Differenzgeschwindigkeit bzw. Relativgeschwindigkeit vorliegt, welche in einen insbesondere mit der gewählten Pulswiederholrate (PRF) eindeutig messbaren Bereich abgebildet wird. In diesem Zusammenhang ist besonders bevorzugt vorgesehen, dass eine sich aus dieser Differenzgeschwindigkeit bzw. Relativgeschwindigkeit ergebende Dopplerfrequenz mehrdeutigkeitsfrei bestimmbar ist.

Nach einer vorteilhaften Ausgestaltung wird vorgeschlagen, dass die Beobachtungsfenstergeschwindigkeit im Wesentlichen der in Schritt a) geschätzten Strömungsgeschwindigkeit entspricht. Der Begriff "im Wesentlichen" umfasst hierbei Abweichungen von maximal 10%. Dies trägt in vorteilhafter Weise dazu bei, eine Differenzgeschwindigkeit bzw. Relativgeschwindigkeit zwischen der Strömungsgeschwindigkeit des Fluids und der Beobachtungsfenstergeschwindigkeit möglichst gering einzustellen, was sich in der Regel vorteilhaft auf eine möglichst mehrdeutigkeitsfreie Darstellung im Dopplerfrequenzspektrum auswirkt. Besonders bevorzugt ist es, wenn die Beobachtungsfenstergeschwindigkeit der in Schritt a) geschätzten Strömungsgeschwindigkeit entspricht.

Nach einer vorteilhaften Ausgestaltung wird vorgeschlagen, dass zum Verschieben des Beobachtungsfensters der zeitliche Abstand zwischen einer Aussendung eines Ultraschall-Impulses und einem (Startzeitpunkt eines) Mess-Zeitintervall von Ultraschall-Impuls zu Ultraschall-Impuls verändert wird. Insbesondere wird zum Verschieben des Beobachtungsfensters und/oder zum Einstellen der Beobachtungsfenstergeschwindigkeit der zeitliche Abstand zwischen einem Aussendezeitpunkt eines Ultraschall-Impulses und einem Anfangszeitpunkt eines Mess-Zeitintervalls von einem Ultraschall-Impuls zu einem (unmittelbar) darauf folgenden Ultraschall-Impuls verändert, insbesondere verlängert oder verkürzt.

Die Position des Beobachtungsfensters (Messbereichs) kann in der Regel (bei bekannter Schallgeschwindigkeit im Fluid) durch Zeitintervalle vorgeben bzw. eingestellt werden. Bei einer Ultraschallmessung werden in der Regel unmittelbar (zeitlich) nach dem Aussenden eines Ultraschallimpulses Reflexionen an Streuern (z. B. Blutkörperchen) direkt vor dem Wandler empfangen. Mit weiter fortschreitender Wellenfront werden anschließend Reflexionen weiter entfernt liegender Bereiche empfangen.

In einem gepulsten Dopplerverfahren werden die empfangenen Signale in der Regel nur in einem bestimmten, zum Aussendezeitpunkt des Ultraschallimpulses zeitlich beabstandeten Zeitintervall verarbeitet. Dies bedeutet mit anderen Worten insbesondere, dass der Anfangszeitpunkt eines einen Anfangszeitpunkt und einen Endzeitpunkt aufweisenden Mess-Zeitintervalls zu einem Aussendezeitpunkt des Ultraschallimpulses zeitlich beabstandet ist und nur diejenigen Reflexionen des ausgesendeten Ultraschallimpulses ausgewertet werden, die in diesem Mess-Zeitintervall wieder empfangen wurden.

Durch die Wahl des zeitlichen Abstandes zwischen dem Aussendezeitpunkt und dem Anfangszeitpunkt des Mess-Zeitintervalls kann der räumliche Abstand des Beobachtungsfensters zum Ultraschallsensor, insbesondere zur Wandlerebene des Ultraschallwandlers gewählt bzw. eingestellt werden. Durch die Länge des Zeitintervalls (zeitlicher Abstand zwischen Anfangszeitpunkt und Endzeitpunkt des Mess-Zeitintervalls) kann die räumliche Ausdehnung des Beobachtungsfensters entlang der Hauptstrahlrichtung des Ultraschallwandlers gewählt bzw. eingestellt werden.

Eine gepulste Dopplermessung besteht in der Regel aus einer Vielzahl einzelner Ultraschallimpulse, d. h. einer schnellen Abfolge von Aussende- und Empfangszeiten mit der Frequenz PRF (Pulse Repetition Frequency). Die PRF ist in diesem Zusammenhang insbesondere die Zeitdauer von Sendepuls zu Sendepuls. Wird der zeitliche Abstand zwischen Aussendezeitpunkt und Anfangszeitpunkt des Mess-Zeitintervalls von Impuls zu Impuls verändert, resultiert dies in einem bewegten Beobachtungsfenster.

Die Beobachtungsfenstergeschwindigkeit kann in diesem Zusammenhang beispielsweise wie folgt eingestellt werden: Wenn der Startzeitpunkt des Aussendens eines Ultraschallimpulses zum Zeitpunkt $t_0$ erfolgt, der Startzeitpunkt der Beobachtung (im Empfänger) zum Zeitpunkt $n \cdot t_{beo,start}$ (wobei n eine natürliche Zahl ist) erfolgt und die Schallgeschwindigkeit $c_0$ im Blut bekannt ist, dann beträgt der Abstand $s_n$ zwischen Ultraschallwandler und Beginn des Beobachtungsfensters:

$$s_n = c_0 \cdot \frac{n \cdot t_{beo,start} - t_0}{2}$$

Bezieht man diesen Abstand nun auf die "Abtastrate" 1/PRF (die Zeit, die zwischen zwei Impulsen vergeht), dann bewegt sich das Beobachtungsfenster mit folgender Geschwindigkeit $v_{beo,start}$ weg vom Ultraschallwandler:

$$v_{beo,start} = \frac{s_n}{1/PRF} = s_n \cdot PRF$$

Nach einer vorteilhaften Ausgestaltung wird vorgeschlagen, dass die Beobachtungsfenstergeschwindigkeit und eine Abtastrate (der Auswerteeinheit (Messeinheit) bzw. des Steuer- und/oder Verarbeitungsgeräts) aneinander angepasst werden. Dies kann in vorteilhafter Weise dazu beitragen, das Signal-zu-Rausch-Verhältnis (kurz: SNR) zu verbessern. Die Abtastrate trägt in diesem Zusammenhang insbesondere zur Auswertung des Empfangssignals bzw. der reflektierten und empfangenen Ultraschallimpulse bei. Besonders vorteilhaft ist es in diesem Zusammenhang, wenn Beobachtungsfenstergeschwindigkeit und eine Abtastrate aneinander nach folgender Gleichung angepasst werden:

$$v_{Gate} = n \cdot \frac{PRF \cdot c_0}{2 \cdot fS} \quad n \in Z$$

Hierbei beschreibt vGate die Beobachtungsfenstergeschwindigkeit, n eine beliebige ganze Zahl, PRF die Pulswiederholrate, c0 die Schallgeschwindigkeit im Fluid und fS die Abtastrate.

Nach einer vorteilhaften Ausgestaltung wird vorgeschlagen, dass die Beobachtungsfenstergeschwindigkeit derart bestimmt wird, dass das Messergebnis der gepulsten Dopplermessung und eine Dopplerverschiebung durch statische Streuer (im Spektrum) voneinander beabstandet sind. Dies erlaubt in vorteilhafter Weise eine Verhinderung der Überdeckung der gesuchten Dopplerfrequenzverschiebung durch den durch statische Streuer, z. B. der Aortenwand, verursachten Frequenzpeak im Dopplerspektrum. Vorzugsweise wird die Beobachtungsfenstergeschwindigkeit derart bestimmt, dass das Messergebnis der gepulsten Dopplermessung und eine Dopplerverschiebung durch statische Streuer im Spektrum nicht aufeinander abgebildet werden und/oder trennbar sind.

Reflexionen durch die nicht beweglichen Streuer, z. B. durch die Aortenwand, werden bei der hier beschriebenen Methode (Verschieben des Beobachtungsfensters) insbesondere nicht mehr bei einer Dopplerfrequenz von 0 Hz dargestellt, sondern werden entsprechend der Geschwindigkeit des Beobachtungsfensters mit einer resultierenden Dopplerfrequenz verschoben. Dadurch kann es zu ungewünschten Überdeckungen bzw. Überlagerungen im DopplerSpektrum kommen, die durch eine insbesondere geringfügige Veränderung der Beobachtungsfenstergeschwindigkeit vorteilhaft vermieden werden kann.

Nach einer vorteilhaften Ausgestaltung wird vorgeschlagen, dass in Schritt c) eine bzw. die Strömungsgeschwindigkeit des Fluids durch eine Addition der Beobachtungsfenstergeschwindigkeit mit einer auf Basis der gepulsten Dopplermessung ermittelten Relativgeschwindigkeit ermittelt wird. Vorzugsweise erfolgt in diesem Zusammenhang eine Ermittlung der tatsächlichen Strömungsgeschwindigkeit des Fluids durch die Addition der bekannten Beobachtungsfenstergeschwindigkeit mit der durch die Messung ermittelten Relativgeschwindigkeit.

Nach einem weiteren Aspekt wird ein implantierbares, vaskuläres Unterstützungssystem vorgeschlagen, eingerichtet zur Durchführung eines hier vorgeschlagenen Verfahrens. Bevorzugt umfasst das Unterstützungssystem ein (elektronisches) Steuer- und/oder Verarbeitungsgerät (Messeinheit), welches zur Durchführung eines hier vorgeschlagenen Verfahrens eingerichtet ist.

Bei dem Unterstützungssystem handelt es sich vorzugsweise um ein linksventrikuläres Herzunterstützungssystem (LVAD) bzw. ein perkutanes, minimalinvasives Linksherz-Unterstützungssystem. Weiterhin bevorzugt ist dieses voll-implantierbar. Das bedeutet mit anderen Worten insbesondere, dass die zur Erfassung erforderlichen Mittel, insbesondere der Ultraschallsensor, sich vollständig im Körper des Patienten befinden und dort verbleiben. Das Unterstützungssystem kann auch mehrteilig bzw. mit mehreren, beabstandet voneinander anordenbaren Komponenten ausgeführt sein, sodass beispielsweise der Ultraschallsensor und ein mit dem Ultraschallsensor verbindbares Steuer- und/oder Verarbeitungsgerät (Messeinheit) des Unterstützungssystems durch ein Kabel separiert voneinander angeordnet sein können. Bei der mehrteiligen Ausführung kann das separat von dem Ultraschallsensor angeordnete Steuer- und/oder Verarbeitungsgerät ebenfalls implantiert oder aber außerhalb des Körpers des Patienten angeordnet werden. Es ist jedenfalls nicht zwingend erforderlich, dass auch die Steuer- und/oder Verarbeitungselektronik im Körper des Patienten angeordnet wird. Beispielsweise kann das Unterstützungssystem so implantiert werden, dass das Steuer- und/oder Verarbeitungsgerät auf der Haut des Patienten bzw. außerhalb des Körpers des Patienten angeordnet wird und eine Verbindung zu dem im Körper angeordneten Ultraschallsensor hergestellt wird. Besonders bevorzugt ist das Unterstützungssystem so eingerichtet bzw. dazu geeignet, dass es zumindest teilweise in einem Ventrikel, bevorzugt dem linken Ventrikel eines Herzens und/oder einer Aorta, insbesondere in Aortenklappenposition angeordnet werden kann.

Weiterhin bevorzugt umfasst das Unterstützungssystem einen Kanal, der vorzugsweise in einem (Einlauf-)Rohr bzw. einer (Einlauf-)Kanüle gebildet ist, eine Strömungsmaschine, wie etwa eine Pumpe und/oder einen Elektromotor. Der Elektromotor ist dabei regelmäßig ein Bestandteil der Strömungsmaschine. Der Kanal ist vorzugsweise so eingerichtet, dass er im implantierten Zustand Fluid aus einem (linken) Ventrikel eines Herzens hin zu der Strömungsmaschine führen kann. Das Unterstützungssystem ist vorzugsweise länglich und/oder schlauchartig gebildet. Bevorzugt sind der Kanal und die Strömungsmaschine im Bereich einander gegenüberliegender Enden des Unterstützungssystems angeordnet.

Es ist insbesondere genau bzw. nur ein Ultraschalsensor vorgesehen. Der Ultraschallsensor weist bevorzugt genau bzw. nur ein Ultraschall-Wandlerelement auf. Dies ist insbesondere dann ausreichend für eine Dopplermessung, wenn dabei das PWD-Verfahren zum Einsatz kommt.

Das in Anspruch 11 angegebene System mit einem implantierbaren, vaskulären Unterstützungssystem und mit einem Steuer- und/oder Verarbeitungsgerät zur Bestimmung mindestens eines Strömungsparameters eines durch das implantierbare, vaskuläre Unterstützungssystem strömenden Fluids umfasst:

a) eine Einrichtung zum Schätzen der Strömungsgeschwindigkeit des Fluids,

b) eine Einrichtung zum Durchführen einer gepulsten Dopplermessung mittels eines Ultraschallsensors in einem Beobachtungsfenster innerhalb des Unterstützungssystems, wobei ein Verschieben des Beobachtungsfensters mit einer Beobachtungsfenstergeschwindigkeit erfolgt, welche unter Verwendung der in Schritt a) geschätzten Strömungsgeschwindigkeit bestimmt wird,

c) eine Einrichtung zum Ermitteln des mindestens einen Strömungsparameters des Fluids unter Verwendung zumindest eines Messergebnisses der gepulsten Dopplermessung oder eines Messergebnisses der gepulsten Dopplermessung und der Beobachtungsfenstergeschwindigkeit.

Die Einrichtung zum Ermitteln des mindestens einen Strömungsparameters des Fluids kann dabei für das Ermitteln einer Strömungsgeschwindigkeit des Fluids unter Verwendung eines Messergebnisses der gepulsten Dopplermessung und der Beobachtungsfenstergeschwindigkeit ausgelegt sein.

Insbesondere kann die Einrichtung zum Schätzen der Strömungsgeschwindigkeit des Fluids für das Schätzen der Strömungsgeschwindigkeit des Fluids auf Basis eines Betriebsparameters einer Strömungsmaschine des Unterstützungssystems ausgelegt sein.

Von Vorteil ist es, wenn die Beobachtungsfenstergeschwindigkeit des Beobachtungsfensters der Einrichtung zum Durchführen einer gepulsten Dopplermessung für das Transformieren einer Dopplerverschiebung in einen mehrdeutigkeitsfrei darstellbaren Bereich ausgelegt ist.

Insbesondere ist es von Vorteil, wenn die Beobachtungsfenstergeschwindigkeit im Wesentlichen einer in der Einrichtung zum Schätzen der Strömungsgeschwindigkeit des Fluids geschätzten Strömungsgeschwindigkeit entspricht.

Die Einrichtung zum Durchführen einer gepulsten Dopplermessung in dem System kann insbesondere für das Verschieben des Beobachtungsfensters durch Verändern des zeitlichen Abstands zwischen einer Aussendung eines Ultraschall-Impulses und einem Mess-Zeitintervall von Ultraschall-Impuls zu Ultraschall-Impuls ausgelegt sein.

Insbesondere kann die Beobachtungsfenstergeschwindigkeit und eine Abtastrate des durch das implantierte, vaskuläre Unterstützungssystem strömenden Fluids aneinander angepasst sein.

Eine vorteilhafte Ausführungsform des Systems sieht vor, dass die Beobachtungsfenstergeschwindigkeit derart bestimmt ist, dass das Messergebnis der gepulsten Dopplermessung und eine Dopplerverschiebung durch statische Streuer voneinander beabstandet sind.

Insbesondere kann vorgesehen sein, dass bei dem System zur Bestimmung mindestens eines Strömungsparameters eines durch ein in den menschlichen Körper implantierbares, vaskuläres Unterstützungssystem strömenden Fluids die Einrichtung zum Ermitteln des mindestens einen Strömungsparameters des Fluids für das Ermitteln der Strömungsgeschwindigkeit des Fluids durch eine Addition der Beobachtungsfenstergeschwindigkeit mit einer auf Basis der gepulten Dopplermessung ermittelten Relativgeschwindigkeit ausgelegt ist.

Die im Zusammenhang mit dem Verfahren erörterten Details, Merkmale und vorteilhaften Ausgestaltungen können entsprechend auch bei dem hier vorgestellten Unterstützungssystem auftreten und umgekehrt. Insoweit wird auf die dortigen Ausführungen zur näheren Charakterisierung der Merkmale vollumfänglich Bezug genommen.

Die hier vorgestellte Lösung sowie deren technisches Umfeld werden nachfolgend anhand der Figuren näher erläutert. Es ist darauf hinzuweisen, dass die Erfindung durch die gezeigten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und/oder Erkenntnissen aus anderen Figuren und/oder der vorliegenden Beschreibung zu kombinieren. Es zeigen schematisch:

Fig. 1: ein implantiertes, vaskuläres Unterstützungssystem in einem Herz,

Fig. 2: ein weiteres implantiertes, vaskuläres Unterstützungssystem in einem Herz,

Fig. 3: einen Ablauf eines hier vorgestellten Verfahrens,

Fig. 4: ein beispielhaftes Doppler-Frequenzspektrum,

Fig. 5: ein weiteres beispielhaftes Doppler-Frequenzspektrum,

Fig. 6: eine Detailansicht eines hier vorgeschlagenen Unterstützungssystems,

Fig. 7: beispielhafte Doppler-Frequenzspektren,

Fig. 8: weitere beispielhafte Doppler-Frequenzspektren,

Fig. 9: weitere beispielhafte Doppler-Frequenzspektren, und

Fig. 10: ein System mit einem implantierbaren, vaskulären Unterstützungssystem und mit einem Steuer- und/oder Verarbeitungsgerät zur Bestimmung mindestens eines Strömungsparameters eines durch das implantierbare, vaskuläre Unterstützungssystem strömenden Fluids.

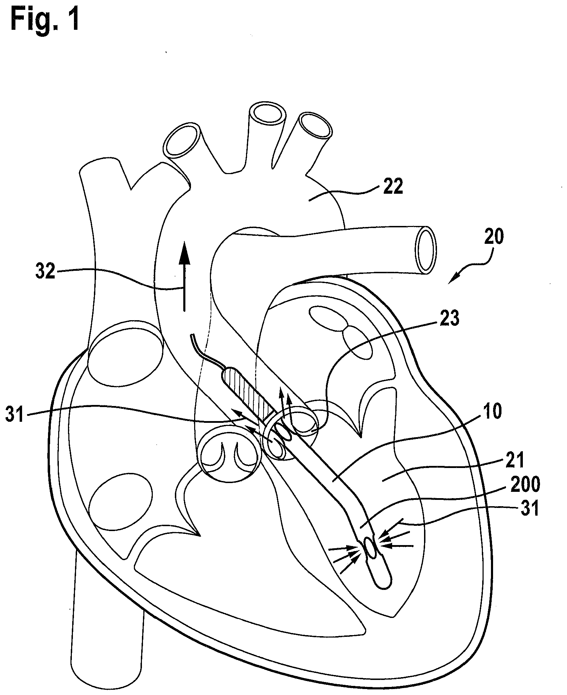

Fig. 1 zeigt schematisch ein implantiertes, vaskuläres Unterstützungssystem 10 in einem Herz 20. Das Unterstützungssystem 10 unterstützt das Herz 20, indem es dazu beiträgt, Blut aus dem (linken) Ventrikel 21 in die Aorta 22 zu fördern. Das Unterstützungssystem 10 wird hierzu in der Aortenklappe 23 verankert, wie Fig. 1 beispielhaft verdeutlicht. Bei einem Unterstützungsgrad von 100% fördert das Unterstützungssystem 10 (LVAD) den kompletten Blutvolumenstrom. Der Unterstützungsgrad beschreibt den Anteil des durch ein Fördermittel, wie etwa eine Pumpe des Unterstützungssystems 10, durch das Unterstützungssystem 10 hindurch geförderten Volumenstroms zum Gesamtvolumenstrom an Blut vom Ventrikel 21 hin zur Aorta 22.

Bei einem Unterstützungsgrad von 100% sind demnach der Fluid-Gesamtvolumenstrom 32 aus dem Ventrikel 21 sowie der Fluid-Volumenstrom 31 durch das Unterstützungssystem 10 identisch. Ein Aortenklappen- bzw. Bypass-Volumenstrom (hier nicht dargestellt; Formelzeichen: $Q_a$) ist folglich dabei Null. Der Fluid-Gesamtvolumenstrom 32 kann auch als (gesamtes) Herz-Zeit-Volumen (HZV, Formelzeichen: $Q_{HZV}$) beschrieben werden. Der Fluid-Volumenstrom 31 kann auch als sog. Pumpenvolumenstrom (Formelzeichen: $Q_p$) bezeichnet werden, der nur den Fluss durch das Unterstützungssystem 10 selbst quantifiziert. Der Unterstützungsgrad kann somit aus dem Verhältnis $Q_p/Q_{HZV}$ berechnet werden.

Bei dem Unterstützungssystem 10 gemäß Fig. 1 handelt es sich beispielhaft um ein Herzunterstützungssystem in Aortenklappenposition. Das Herzunterstützungssystem 10 ist in einem Herz 20 platziert. Blut wird aus dem Ventrikel 21 entnommen und in die Aorta 22 abgegeben. Durch die Operation des Herzunterstützungssystems 10 (Pumpenteil) entsteht ein Blutstrom 31.

Bei Unterstützungssystemen 10 nach Bauart von Fig. 1 wird das Blut im Inneren eines kanülenartigen Abschnitts bzw. Kanals 200 des (Herz-)Unterstützungssystems 10 durch die Aortenklappe 23 hindurch gefördert und im Bereich der Aorta 22 wieder abgegeben. Zur Integration eines Ultraschallwandlers bietet sich hier besonders bevorzugt die (in den Ventrikel 21 hinein ragende) Spitze des Unterstützungssystems 10 an, sodass in diesem Fall das Blut vom Ultraschallwandler weg in den kanülenartigen Abschnitt bzw. Kanal 200 des (Herz-)Unterstützungssystems 10 wegströmt.

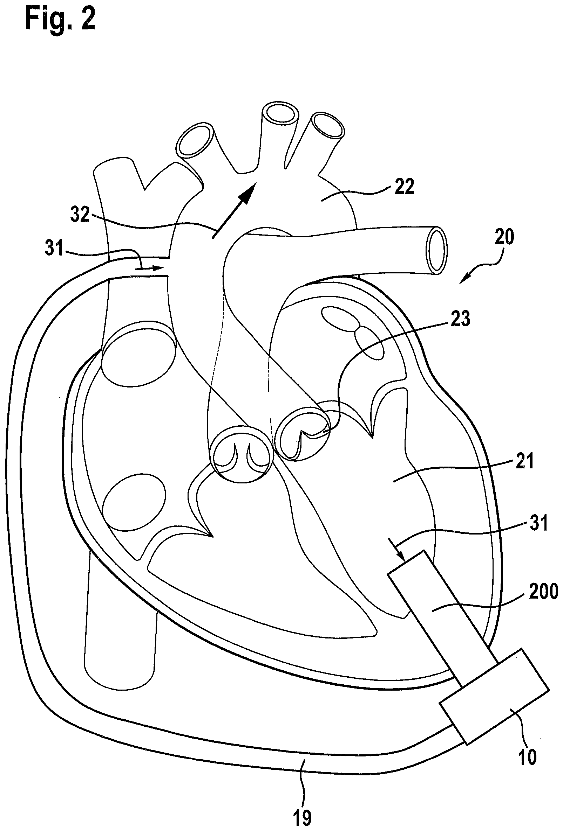

Fig. 2 zeigt schematisch ein weiteres implantiertes, vaskuläres Unterstützungssystem 10 in einem Herz 20.

Bei dem Unterstützungssystem 10 gemäß Fig. 2 handelt es sich beispielhaft um ein Herzunterstützungssystem in apikaler Position. Die Bezugzeichen werden einheitlich verwendet, sodass auf die vorangehenden Ausführungen zur Fig. 1 vollumfänglich Bezug genommen werden kann.

Bei Unterstützungssystemen 10 nach Bauart von Fig. 2 wird das Blut durch einen kanülenartigen Abschnitt bzw. Kanal 200 angesaugt und durch einen Bypass 19 außerhalb des Herzens 20 in die Aorta 22 zurückgeführt. Hierbei bietet sich die Integration eines Ultraschallwandlers im Pumpengehäuse des (Herz-)Unterstützungssystems 10, mit Blick aus dem ansaugenden kanülenartigen Abschnitt 200 in Richtung Ventrikel 21 heraus, an. Dies bedeutet mit anderen Worten insbesondere, dass der Ultraschallwandler in dem Unterstützungssystem 10 angeordnet, sowie zu dem Kanal 200 und zu dem Ventrikel 21 hin ausgerichtet ist. In diesem Fall strömt das Blut auf den Ultraschallwandler zu. Das hier vorgeschlagene Verfahren funktioniert mit beiden Varianten nach Fig. 1 und Fig. 2 gleichermaßen, da lediglich die Bewegungsrichtung des Messfensters (etwa in einem Computerprogramm) angepasst werden muss.

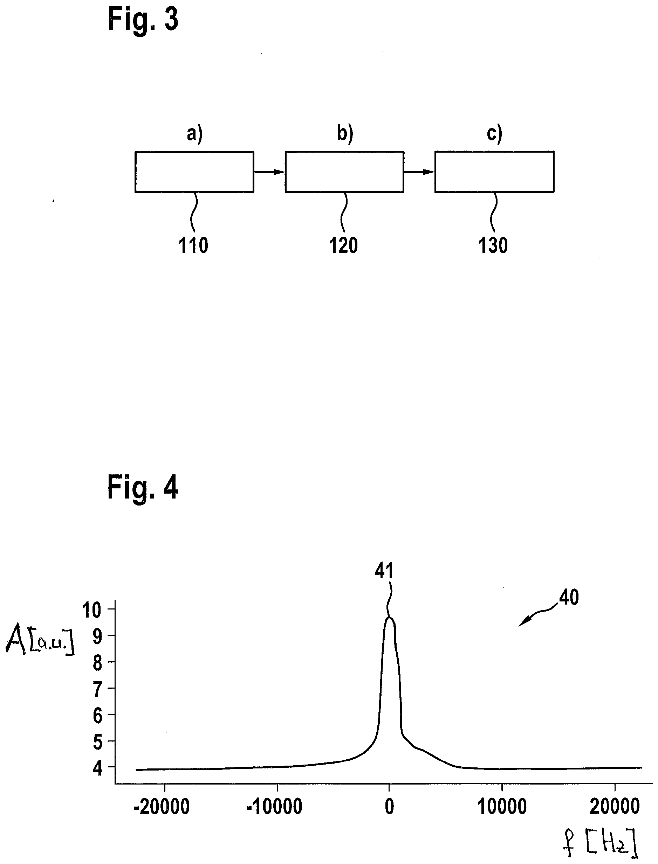

Fig. 3 zeigt schematisch einen Ablauf eines hier vorgestellten Verfahrens in einem System zur Bestimmung mindestens eines Strömungsparameters eines durch ein implantierbares, vaskuläres Unterstützungssystems strömenden Fluids.

Das Verfahren dient zur Bestimmung einer Strömungsgeschwindigkeit eines durch ein implantiertes, vaskuläres Unterstützungssystem strömenden Fluids. Die dargestellte Reihenfolge der Verfahrensschritte a), b) und c) mit den Blöcken 110, 120 und 130 ist lediglich beispielhaft und kann sich so bei einem regulären Betriebsablauf einstellen. In Block 110 erfolgt ein Schätzen der Strömungsgeschwindigkeit des Fluids. In Block 120 erfolgt ein Durchführen einer gepulsten Dopplermessung mittels eines Ultraschallsensors des Unterstützungssystems in einem Beobachtungsfenster innerhalb des Unterstützungssystems, wobei ein Verschieben des Beobachtungsfensters mit einer Beobachtungsfenstergeschwindigkeit erfolgt, welche unter Verwendung der in Schritt a) geschätzten Strömungsgeschwindigkeit bestimmt wird. In Block 130 erfolgt ein Ermitteln des mindestens einen Strömungsparameters des Fluids unter Verwendung zumindest eines Messergebnisses der gepulsten Dopplermessung oder eines Messergebnisses der gepulsten Dopp-lermessung und der Beobachtungsfensterge schwindigkeit.

Für eine exemplarische Darstellung des Verfahrens werden folgende Parameter angenommen:

- Durchmesser Einlauf- bzw. Messbereich z. B. 5 mm,
- Maximal zu messender Blutfluss z. B. Q = 9 l/min,
- Resultierende max. Blutflussgeschwindigkeit: $v_{Blut,max}$ = 7,64 m/s,
- Schallgeschwindigkeit im Blut z. B. $c_{Blut}$ = 1540 m/s,
- Ultraschallfrequenz z. B. $f_0$ = 6 MHz,
- Abstand Ultraschallelement zu Beginn Betrachtungsfenster z. B. 25 mm,
- Anzahl Ultraschall-Schwingungszyklen pro ausgesendetem Ultraschall-PWD-Impuls z. B. 10,
- Resultierende Länge des durch den Ultraschallimpuls erzeugten Wellenpakets (in Strecke): $l_{Burst} = c_0 \times 10/f_0$ = 2,57 mm,
- Resultierende maximale Ausbreitungsstrecke Ultraschall-Impulses: d = 55,13 mm.

Aus diesen Angaben ergibt sich für eine Messung direkt in Abstrahlrichtung (Flussrichtung entspricht Haupt-abstrahlrichtung; $\alpha$ = 0) folgende (zu erwartende) maximale Doppler-Verschiebung:

$$df = \frac{2 \cdot v_{Blut,max} \cdot f_0}{c_0} = \frac{2 \cdot 7,64\frac{m}{s} \cdot 6MHz}{1540\frac{m}{s}} = 59,53kHz \tag{1}$$

Die Messung soll als gepulste Dopplermessung ausgeführt werden, bei welcher ein neuer Ultraschall-Impuls erst ausgesendet wird, wenn alle signifikanten Echos eines unmittelbar zuvor ausgesendeten Ultraschall-Impulses abgeklungen sind. Die Wahl der hierfür zu verwendenden Pulswiederholrate (PRF) wird nachstehend erläutert.

Unter Beachtung des (Nyquist-)Abtasttheorems (welches hier jedoch gerade nicht beachtet werden muss bzw. erst durch das Verschieben des Beobachtungsfensters erfüllbar wird, da nur die Relativgeschwindigkeit zwischen Fluid und Beobachtungsfenster erfasst werden muss) würde eine maximale Dopplerfrequenz von 59,53 kHz bei reellwertiger Auswertung bedeuten, dass eine minimale Pulswiederholrate bzw. minimale Pulswiederholfrequenz von

$$PRF_{min} = 2 \cdot df = 119,06kHz \, . \tag{2}$$

eingestellt werden müsste.

Bei den hier im Fokus stehenden implantierten, vaskulären Unterstützungssystemen ergibt sich jedoch aus der geometrischen Betrachtung (maximale Ausbreitungsstrecke des Ultraschallimpulses) bzw. den geometrischen Randbedingungen im Unterstützungssystem und der daraus resultierenden Laufzeit aller relevanten Signalanteile die folgende maximale Pulswiederholrate PRFmax.

$$PRF_{max} = \frac{c_{Blut}}{d} = 27,93 kHz \qquad (3)$$

Somit ist die maximale Pulswiederholrate der gepulsten Dopplermessungen hier (bzw. für die im Fokus stehenden Unterstützungssysteme) kleiner als das Zweifache der maximal auftretenden Doppler-Verschiebung.

Diese Randbedingungen führen zu einer Verletzung des Abtasttheorems und folglich zu einer Mehrdeutigkeit der Messergebnisse, die durch eine Auswertung bzw. Methode (Verschieben des Beobachtungsfensters), wie in den folgenden Abschnitten beschrieben, behoben werden kann.

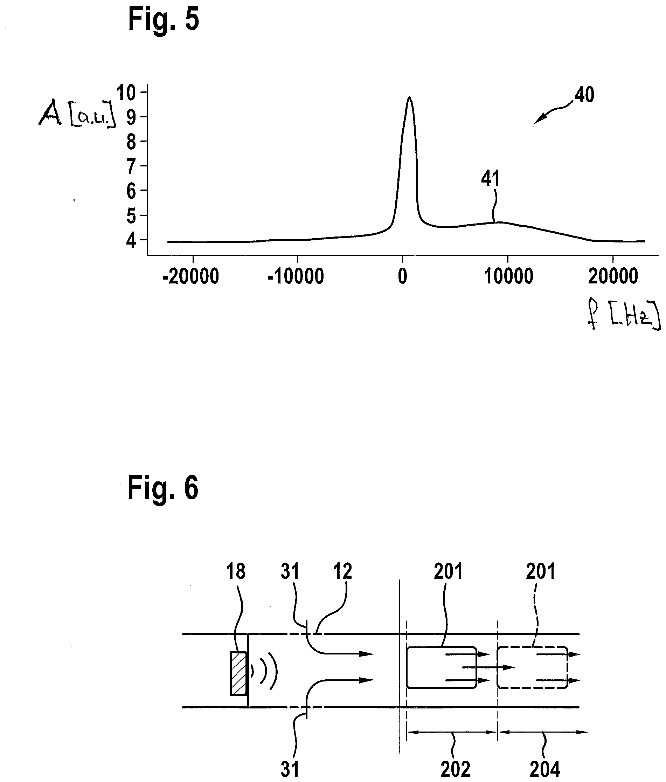

Zuvor wird jedoch zur Veranschaulichung der bei diesen Randbedingungen entstehenden Problematik die dabei auftretende Mehrdeutigkeit in den Fig. 4 und 5 veranschaulicht (die mit der hier vorgestellten Methode vorteilhaft vermieden werden kann). Fig. 4 zeigt schematisch ein beispielhaftes Doppler-Frequenzspektrum 40. Fig. 4 zeigt eine Doppler-Verschiebung bei einem Volumenstrom von 3 l/min und einer Pulswiederholrate von ca. 25 kHz. Die Hauptfrequenzkomponente 41 (Peak) liegt unter der Trägerfrequenz bei ca. 0 Hz. Fig. 5 zeigt schematisch ein weiteres beispielhaftes Doppler-Frequenzspektrum 40. Fig. 5 zeigt eine Doppler-Verschiebung bei einem Volumenstrom von 3 l/min und einer Pulswiederholrate von ca. 20 kHz. Die Hauptfrequenzkomponente 41 (Peak) liegt bei ca. +8 kHz. Dies veranschaulicht insbesondere, dass bei unterschiedlichen PRFs und dem identischen Volumenstrom unterschiedliche Frequenzen ausgegeben werden und dadurch ohne die Verwendung der hier beschriebenen Erfindung ein durch die Pumpe eingestellter Volumenstrom nicht eindeutig bestimmt werden können. Beispielsweise bei 20 kHz PRF befindet sich der Peak bei 3 l/min bei einer Frequenz von ca. 8 KHz, was insbesondere einer Geschwindigkeit von 0,77 m/s bzw. einem Volumenstrom von 0,9 l/min entspräche. Der tatsächlich (zu messende) Volumenstrom beträgt bei diesem Beispiel aber 3 l/min. Diese Messungen wurden außerdem bei 8 MHz Ultraschall-Frequenz durchgeführt.

In den nachfolgenden Abschnitten wird eine beispielhafte Methode im Sinne der hier vorgeschlagenen Lösung, beschrieben, bei der entsprechende, mehrdeutige Messergebnisse vorteilhaft vermieden werden können.

Hierzu wird vorgeschlagen, das Beobachtungsfenster mit einer Beobachtungsfenstergeschwindigkeit zu verschieben, welche unter Verwendung einer geschätzten Strömungsgeschwindigkeit des Fluids (hier des Blutes) bestimmt wird. Dies erlaubt in vorteilhafter Weise, die Dopplerverschiebung in einen Bereich zu transformieren, der mit der gewählten Ultraschallfrequenz und PRF mehrdeutigkeitsfrei darstellbar ist. Besonders vorteilhaft ist es im Zusammenhang mit der Verschiebung des Beobachtungsfensters, wenn die radialen Querschnitte der Blutflussgeschwindigkeiten über einen gewissen Bereich (einige Zentimeter) in axialer Verlängerung zum Ultraschallelement unverändert sind. Die beschriebene Methode ist in Herzunterstützungssystemen verschiedener Bauart verwendbar, beispielsweise in Systemen in Aortenklappenposition, wie beispielhaft in Fig. 1 dargestellt, oder beispielsweise auch bei apikal platzierten Systemen, wie beispielhaft in Fig. 2 dargestellt.

Eine ultraschallbasierte Pumpenvolumenstrommessung basiert in der Regel auf ein oder mehreren in das Unterstützungssystem integrierten Ultraschallwandlern und einem gegebenenfalls räumlich abgesetzten (elektronischen) Steuer- und/oder Verarbeitungsgerät, das auch als Messeinheit bezeichnet werden kann. Das räumlich abgesetzte Steuer- und/oder Verarbeitungsgerät kann sowohl implantiert platziert als auch durch eine transkutane Leitung verbunden extrakorporal platziert sein. Es bildet dann mit dem implantierbaren, vaskulären Unterstützungssystem ein System zur Bestimmung mindestens eines Strömungsparameters des durch das implantierbare, vaskuläre Unterstützungssystem strömenden Fluids.

Die beschriebenen Ausführungsformen nach Fig. 1 und Fig. 2 erfordern insbesondere ein gepulstes Dopplermessverfahren (pulsed wave Doppler), um das Beobachtungsfenster (den Messbereich bzw. das Messfenster) entlang der Hauptstrahlrichtung des Ultraschallwandlers positionieren zu können. Aufgabe des Steuer- und/oder Verarbeitungsgeräts bzw. der Messeinheit ist die Erzeugung geeigneter Ultraschallimpulse zur Abstrahlung durch den oder die Ultraschallwandler, Empfang und Verstärkung empfangener gestreuter Ultraschallenergie (Reflexionen, Echos), sowie eine Verarbeitung der empfangenen Signale zur Berechnung eines Doppler-Frequenzspektrums.

Die Wahl der Position des Beobachtungsfensters erfolgt dabei aufgrund der ausreichend bekannten Schallgeschwindigkeit im Blut in der Regel durch Zeitintervalle. Nach dem Aussenden eines Ultraschallimpulses werden sofort Reflexionen an Streuern (z. B. Blutkörperchen) direkt vor dem Wandler empfangen. Mit weiter fortschreitender Wellenfront werden anschließend Reflexionen weiter entfernt liegender Bereiche empfangen. In einem gepulsten Dopplerverfahren werden die empfangenen Signale nur in einem bestimmten zum Aussendezeitpunkt des Ultraschallimpulses zeitlich beabstandeten Zeitintervalls verarbeitet.

Durch die Wahl des zeitlichen Abstandes kann der räumliche Abstand des Beobachtungsfensters zur Wandlerebene des Ultraschallwandlers gewählt bzw. eingestellt werden. Durch die Länge des Zeitintervalls kann die räumliche Ausdehnung des Beobachtungsfensters entlang der Hauptstrahlrichtung des Ultraschallwandlers gewählt bzw. eingestellt werden.

Eine gepulste Dopplermessung besteht in der Regel aus einer Vielzahl einzelner Ultraschallimpulse, d. h. einer schnellen Abfolge von Aussende- und Empfangszeiten mit der Frequenz PRF (Pulse Repetition Frequency). Die PRF ist in diesem Zusammenhang insbesondere die Zeitdauer von Sendepuls zu Sendepuls. Wird der zeitliche Abstand zwischen Aussendung und Mess-Zeitintervall von Impuls zu Impuls verändert, resultiert dies in einem bewegten Beobachtungsfenster. Das bedeutet mit anderen Worten auch, dass zum Verschieben des Beobachtungsfensters der zeitliche Abstand zwischen einer Aussendung eines Ultraschall-Impulses und einem Startpunkt eines Mess-Zeitintervalls von Ultraschall-Impuls zu Ultraschall-Impuls verändert werden muss.

Fig. 6 zeigt schematisch eine Detailansicht eines hier vorgeschlagenen Unterstützungssystems 10. Die Darstellung nach Fig. 6 betrifft dabei einen exemplarischen Aufbau eines Herzunterstützungssystems 10, in dem ein hier vorgeschlagenes Verfahren angewendet werden kann.

Das Ultraschallelement 18 repräsentiert hier den Ultraschallsensor 18 und strahlt in Richtung der Blutflussgeschwindigkeit ab. Im Bereich eines (mit Öffnungen versehenen) Einlaufkäfigs 12 des Unterstützungssystems 10 zeigt das einströmende Blut 31 noch kein gleichbleibendes Strömungsprofil. Im weiteren Verlauf stromabwärts in den Bereichen 202 und 204 ist das radiale Strömungsprofil jedoch weitgehend konstant. Somit kann das Beobachtungsfenster 201 vorteilhaft in diesem Bereich mit der Beobachtungsfenstergeschwindigkeit $v_{Gate}$ verschoben werden. Die Bereiche 202 und 204 können bei den in Fig. 1 und Fig. 2 gezeigten Ausführungsformen beispielsweise in dem Kanal 200 liegen.

Wenn zum Beispiel, wie in nachfolgender Gleichung (4) gezeigt, bei einer PRF von 25 kHz und einer Ultra-schallfrequenz von $f_0$ = 4 MHz, eine Strömungsgeschwindigkeit von $v_{Blut}$ = 3 m/s weg vom Piezo-Element des Ultraschallsensors 18 in einem fixiertem Beobachtungsfenster gemessen werden soll, führt dies zu einer Dopplerverschiebung von - 15,58 kHz. Diese Dopplerverschiebung kann bei der gegebenen PRF von 25 kHz und der Auswertung von positiven und negativen Geschwindigkeiten nicht mehr im negativen Teil des Dopplerspektrums dargestellt werden und wird dementsprechend als 9,42 kHz in dem positiven Frequenzbereich des Spektrums dargestellt.

Wenn allerdings das Beobachtungsfenster 201 (wie hier vorgeschlagen) mit einer Verschiebegeschwindigkeit von beispielhaft $v_{Gate}$ = 1,75 m/s weg vom Piezo-Element des Ultraschallsensors 18 bewegt wird, wird die resultierende (bzw. relative), zu transformierende Strömungsgeschwindigkeit verringert, hier beispielhaft auf 3 m/s - 1,75 m/s = 1,25 m/s reduziert. Die daraus resultierende Dopplerverschiebung von - 6,49 kHz ist bei einer PRF von 25 kHz mehrdeutigkeitsfrei im Dopplerspektrum darstellbar (siehe nachfolgende Gleichung (7)).

$$f_{d,wrapped} = \frac{-2 \cdot v_{Blut} \cdot f_0}{c_0} \tag{4}$$

$$= \frac{-2 \cdot 3\frac{m}{s} \cdot 4MHz}{1540\frac{m}{s}} \tag{5}$$

$$= -15,58 kHz \tag{6}$$

$$f_{d,tracking_{doppl}} = \frac{-2 \cdot (v_{Blut} - v_{Gate}) \cdot f_0}{c_0} \tag{7}$$

$$= \frac{-2 \cdot (3\frac{m}{s} - 1,75\frac{m}{s}) \cdot 4MHz}{1540\frac{m}{s}} \tag{8}$$

$$= -6,49 kHz \tag{9}$$

Dies stellt ein Beispiel dafür dar, dass und wie die Beobachtungsfenstergeschwindigkeit so bestimmt werden kann, dass eine Dopplerverschiebung in einen Bereich transformiert wird, der mehrdeutigkeitsfrei darstellbar ist.

Eine Basis für eine entsprechende Bestimmung der Beobachtungsfenstergeschwindigkeit bildet hier insbesondere eine zuvor vorgenommene Schätzung der Strömungsgeschwindigkeit des Blutes durch das Unterstützungssystem. Vorteilhafterweise fußt diese Schätzung auf einer zuvor durchgeführten Ultraschallmessung (z. B. mit festem Beobachtungsfenster) mittels des Ultraschallsensors 18 des Unterstützungssystems 10. Dies ist jedoch nur beispielhaft. So könnte die Schätzung z. B auch auf einem Erfahrungswert, beispielsweise basierend auf dem Alter und/oder ggf. der Schwere der Erkrankung des Patienten beruhen.

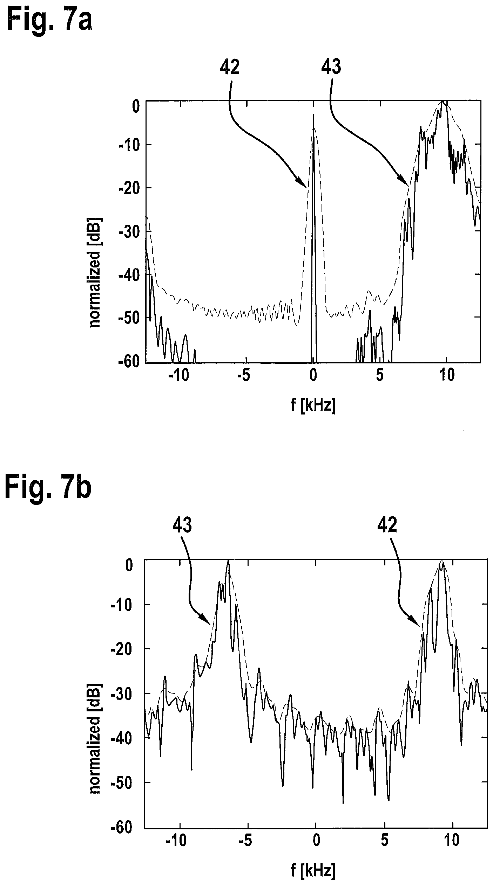

Fig. 7 zeigt schematisch beispielhafte Doppler-Frequenzspektren. Die dargestellten Doppler-Frequenzspektren können sich beispielsweise bei einem Einsatz der hier vorgestellten Methode ergeben.

Fig. 7 veranschaulicht Dopplerspektren bei einer Blutflussgeschwindigkeit von 3 m/s bei einer Ultraschallfrequenz von 4 MHz mit einem unfokussierten Piezo-Element von 6 mm Durchmesser und einer PRF von 25 kHz. Fig. 7a veranschaulicht das gealiaste (mehrdeutigkeitsbehaftete) Dopplerspektrum einer Messung mit einem Beobachtungsfenster in einem festen Abstand von 25 mm vom Piezo-Element. Fig. 7b veranschaulicht demgegenüber das nicht gealiaste (mehrdeutigkeitsfreie) Dopplerspektrum bei einem von 15 mm bis 25 mm vom Piezo-Element verschobenen Beobachtungsfenster bei einer Verschiebegeschwindigkeit (Beobachtungsfenstergeschwindigkeit) von 1,75 m/s.

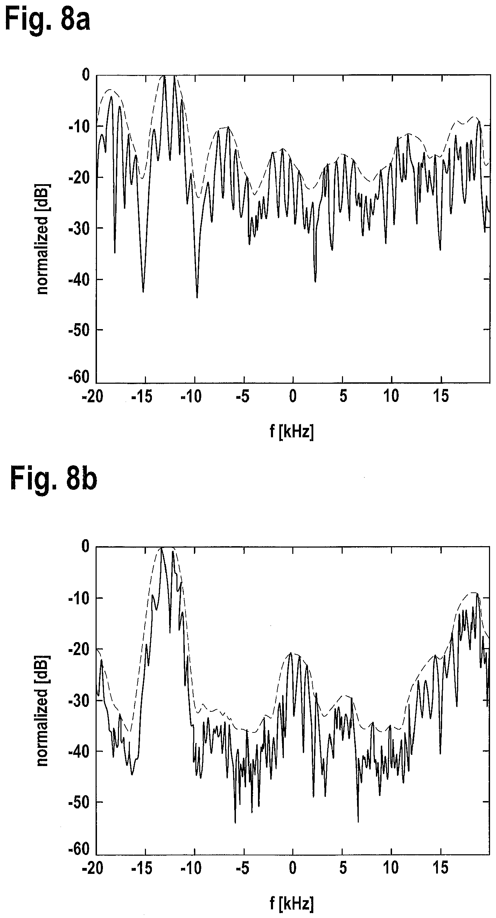

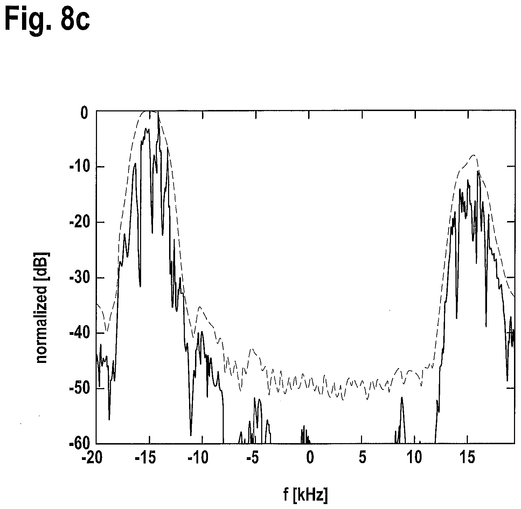

Weiterhin sind in den Dopplerspektren gemäß Fig. 7 jeweils zwei Ausschläge bzw. Peaks zu erkennen, nämlich ein Peak, der auf die durch die Aortenwand verursachte Dopplerverschiebung (unbewegter Streuer) 42 zurückgeht, und ein Peak, der durch die Reflexion an bewegten Streuern (z. B. Blutkörperchen) 43 hervorgerufen wird. In den Fig. 7 und 8 beschreiben die durchgezogenen Linien Ergebnisse einer Fourier-Transformation und die gestrichelten Linien Ergebnisse der sog. Weich-Methode.

Fig. 7 veranschaulicht, wie durch die hier beschriebene Methode aliasing verhindert werden kann. Fig. 7b zeigt weiterhin, wie durch die Verschiebung des Beobachtungsfensters auf der rechten Seite des Spektrums ein zweiter Peak 42 außerhalb von 0 Hz entsteht. Dieser Peak 42 (der z. B. die Dopplerverschiebung des unbewegten Streuers Aortenwand beschreibt) resultiert aus der relativen Bewegung des Beobachtungsfensters zum ortsfesten Gewebe z. B. der Aortenwand und zeigt somit die Dopplerfrequenz des Beobachtungsfensters bzw. die Dopplerfrequenz, welche die Beobachtungsfenstergeschwindigkeit betrifft.

In Fig. 7b ist auch zu erkennen, dass sich die Peakbreiten der beiden Peaks (im Vergleich zu dem Peakbreiten in Fig. 7a) durch das bewegte Beobachtungsfenster ändern. Der Peak 42, der durch die Reflexion an ortsfestem Gewebe der Aortenwand entsteht, verbreitert sich. Der Peak 43, der durch mit der Blutflussgeschwindigkeit bewegte Streuer (wie z. B. Blutkörperchen) entsteht, wird demgegenüber schmaler.

Fig. 8 zeigt schematisch weitere beispielhafte Doppler-Frequenzspektren. Die dargestellten Doppler-Frequenzspektren können sich beispielsweise bei einem Einsatz der hier vorgestellten Methode ergeben.

Die Verschlechterung des Signal-zu-Rausch-Verhältnisses (kurz: SNR), die in Fig 7 zu erkennen ist, ist eine Folge einer Fehlanpassung der Beobachtungsfenstergeschwindigkeit an die Abtastrate des Empfangssignals, wodurch das Beobachtungsfenster jittert. Eine Reduktion des Jitterns und dadurch eine Verbesserung des SNR im Spektrum, kann beispielsweise durch eine Anpassung der Abtastfrequenz an die Beobachtungsfenstergeschwindigkeit, ein Resampling des Empfangssignals und/oder eine Überabtastung erreicht werden.

Nachstehende Gleichung (10) zeigt, wie die Beobachtungsfenstergeschwindigkeit des Beobachtungsfensters und die Abtastrate besonders vorteilhaft aneinander angepasst werden können. Fig. 8 veranschaulicht die genannten Möglichkeiten zur Verbesserung des SNR.

Gleichung 10 zeigt, wie die Geschwindigkeit des Beobachtungsfensters bei gegebener Schallgeschwindigkeit im Blut $c_0$, gegebener PRF und gegebener Abtastrate $f_S$ besonders vorteilhaft gewählt werden kann, um das SNR zu maximieren.

$$v_{Gate} = n \cdot \frac{PRF \cdot c_0}{2 \cdot f_S} \quad n \in Z \qquad (10)$$

Dies stellt ein Beispiel dafür dar, dass und ggf. wie die Beobachtungsfenstergeschwindigkeit und eine Abtastrate aneinander angepasst werden können.

Die Fig. 8a, 8b und 8c zeigen jeweils ein Dopplerspektrum, nach Anwendung der hier beschriebenen Methode, bei einer Flussgeschwindigkeit von 3 m/s, bei der Verwendung eines nicht fokussierten Piezo-Elements mit einem Durchmesser von 4 mm, einer Ultraschallfrequenz von 8 MHz und einer PRF von 40 kHz. Das Beobachtungsfenster bewegt sich bei den Messungen, deren Ergebnis in den Fig. 8a, 8b und 8c veranschaulicht ist, jeweils von einem Abstand von 15 mm bis zu einem Abstand von 30 mm vom Piezo-Element.

Bei den Fig. 8a und 8b bewegte sich das Beobachtungsfenster mit einer (Beobachtungsfenster-)Geschwindigkeit von 1,75 m/s. Bei Fig. 8a wurde eine Abtastrate von 20 MHz verwendet und bei Fig. 8b eine Abtastrate von 100 MHz. Fig. 8c veranschaulicht das SNR bei aufeinander angepasster Abtastrate von 20 MHz und einer Verschiebegeschwindigkeit des Beobachtungsfensters von 1,54 m/s.

Bei der Anwendung des hier beschriebenen Verfahrens kann in vorteilhafter Weise ein weiteres Ziel erreicht werden, nämlich die Reduktion der spektralen Verbreiterung des gesuchten Frequenzpeaks bei hohen Blutflussgeschwindigkeiten. Bei einer Verwendung von Auswerteverfahren (mit festem Beobachtungsfenster), die nicht nach der hier beschriebenen Methode arbeiten, kann dieser zusätzliche Effekt in der Regel nicht erreicht werden. Auf der Basis dieser schmaleren, durch die Flussgeschwindigkeit des Blutes verursachten Frequenzpeaks im Dopplerspektrum, kann die Genauigkeit der Ermittlung (Schätzung) der Hauptgeschwindigkeitskomponente deutlich verbessert werden.

Durch die Verschiebung des Beobachtungsfensters mit $v_{Gate}$, der grob geschätzten Flussgeschwindigkeit der bewegten Streuer (wie etwa Blutkörperchen) im Blut, wird die Verweildauer für alle bewegten Streuer, für die $|v_{Blut} - v_{Gate}| < v_{Blut}$ gilt, im Beobachtungsfenster verlängert. Dies kann in vorteilhafter Weise durch den Integrationsgewinn bei der anschließenden Fourier Transformation zu einer Verbesserung des SNR (Amplitude) von $\sqrt{(N)}$ führen, wobei N der Anzahl der Abtastungen entspricht, während sich der Streuer im Beobachtungsfenster befindet.

Für die statischen Streuer, z. B. der Aortenwand, für welche die Bedingung $|v_{Blut} - v_{Gate}| < v_{Blut}$ nicht erfüllt ist, bewegen sich die Streuer nicht mehr wie bei einer herkömmlichen Auswertung während der gesamten Beobachtungsdauer im Beobachtungsfenster. Durch die Verwendung der hier beschriebenen Methode wird diese Dauer, insbesondere abhängig von der Flussgeschwindigkeit des Bluts bzw. der Geschwindigkeit des Beobachtungsfensters, signifikant verkürzt. Dies kann mit anderen Worten auch wie folgt beschrieben werden: Bei einem ortsfesten Fenster und "einem" ortsfesten Streuer wird der gesamte Wellenzug an diesem reflektiert. Also wenn die Beobachtungdauer kleiner/gleich der Impulsdauer des Wellenzugs gewählt ist, wird an diesem während der gesamten Beobachtungszeit ein Teil des Impulses reflektiert. Diese lange Verweildauer im Beobachtungsfenster (Zeitbereich) liefert einen schmalbandigen Peak im Spektrum (Frequenzbereich). Durch das Bewegen des Fensters wird die Verweildauer verkürzt und der Peak im Spektrum breitbandiger. Die dadurch resultierende Reduktion des Integrationsgewinns (im Vergleich zu bekannten Verfahren) führt, wie in Fig. 7 gezeigt, zu einer Verbreiterung des durch statische Streuer (nun nicht mehr bei 0 Hz gelegenen) hervorgerufenen Frequenzpeaks und zu einer Verschmierung der Signalenergie im Spektrum.

Ein zusätzlicher besonderer Vorteil der hier beschriebenen Methode ist, dass die Verschiebegeschwindigkeit des Beobachtungsfensters (Beobachtungsfenstergeschwindigkeit) in einem gewissen Rahmen frei gewählt werden kann. Tritt beispielsweise der Fall auf, dass die mit $v_{Gate}$ beobachteten, statischen Streuer, die eine Dopplerverschiebung von

$$f_{d,stat} = \frac{2 \cdot v_{Gate} \cdot f_0}{c_0} \qquad (11)$$

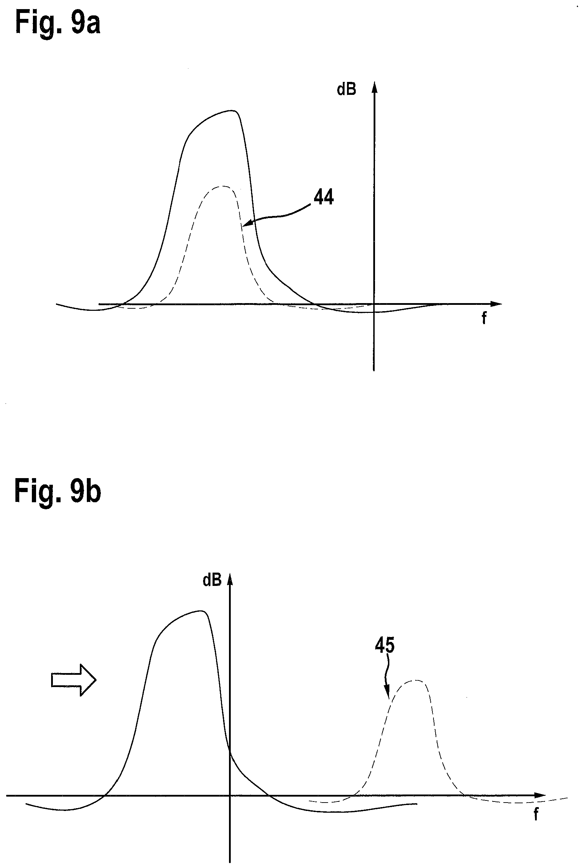

erfahren, im selben Frequenzbereich liegen wie die gesuchte Dopplerverschiebung durch den Blutfluss (es werden keine zwei sondern nur ein Peak im Spektrum detektiert), so kann die Verschiebegeschwindigkeit des Beobachtungsfensters in vorteilhafter Weise leicht verändert werden, sodass der gesuchte Frequenzpeak nicht mehr vom wesentlich stärkeren Frequenzpeak durch statische Streuer überdeckt ist. Dieser Effekt ist in Fig. 9 schematisch dargestellt. Die Verringerung der spektralen Verbreiterung ist in dieser schematischen Darstellung nicht berücksichtigt.

Fig. 9 zeigt schematisch weitere beispielhafte Doppler-Frequenzspektren. Die dargestellten Doppler-Frequenzspektren können sich beispielsweise bei einem Einsatz der hier vorgestellten Methode ergeben.

Durch eine leichte Veränderung der Verschiebegeschwindigkeit $v_{Gate}$ des Beobachtungsfensters kann die Überdeckung des gesuchten, durch die Blutflussgeschwindigkeit verursachten Frequenzpeaks durch den durch statische Streuer verursachten Frequenzpeak behoben werden.

In Fig. 9a ist die gesuchte Dopplerfrequenz der Flussgeschwindigkeit 44 überdeckt. In Fig. 9b ist die gesuchte Dopplerfrequenz der Flussgeschwindigkeit 44 nicht mehr überdeckt. Hierzu fand eine geringfügige Veränderung der Verschiebegeschwindigkeit des Beobachtungsfensters (Beobachtungsfenstergeschwindigkeit) statt. Dies stellt ein Beispiel dafür dar, dass und ggf. wie die Beobachtungsfenstergeschwindigkeit derart bestimmt werden kann, dass das Messergebnis der gepulsten Dopplermessung und eine Dopplerverschiebung durch statische Streuer voneinander beabstandet sind.

Die hier vorgestellte Lösung ermöglich insbesondere einen oder mehrere der nachfolgenden Vorteile:

- PWD-basierte Flussgeschwindigkeits- bzw. Volumenflussmessung wird auch bei großem Abstand zwischen Messfenster und Ultraschallwandler ermöglicht.
- Auflösung der geometrisch bedingten Mehrdeutigkeit der Dopplerverschiebung aufgrund von geometrischen Randbedingungen im Unterstützungssystem (VAD).
- Reduktion der spektralen Verbreiterung.
- Erhöhung der Genauigkeit der Dopplerfrequenzschätzung.
- Genauere Bestimmung der Flussgeschwindigkeit.
- Verhinderung der Überdeckung der gesuchten Dopplerfrequenzverschiebung durch den durch statische Streuer, z. B. der Aortenwand, verursachten Frequenzpeak im Dopplerspektrum.

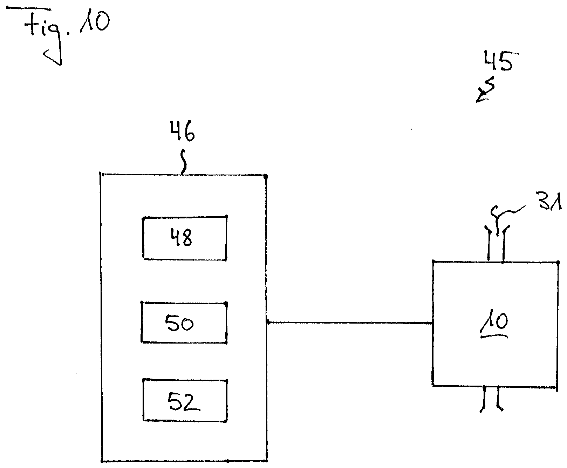

Das in der Fig. 10 gezeigte System 45 umfasst ein implantierbares, vaskuläres Unterstützungssystem 10 und enthält ein Steuer- und/oder Verarbeitungsgerät 46 zur Bestimmung mindestens eines Strömungsparameters eines durch das implantierbare, vaskuläre Unterstützungssystem 10 strömenden Fluids 31. Das Steuer- und/oder Verarbeitungsgerät 46 ist mit dem implantierbaren, vaskulären Unterstützungssystem 10 durch eine transkutane Leitung verbunden und kann extrakorporal platziert werden. Zu bemerken ist, dass das Steuer- und/oder Verarbeitungsgerät 46 grundsätzlich auch für das implantierte Platzieren im menschlichen Körper ausgelegt sein kann.

In dem Steuer- und/oder Verarbeitungsgerät 46 gibt es eine Einrichtung 48 zum Schätzen der Strömungsgeschwindigkeit des Fluids 31. Das Steuer- und Verarbeitungsgerät 46 enthält eine Einrichtung 50 zum Durchführen einer gepulsten Dopplermessung mittels eines in der Fig. 6 gezeigten Ultraschallsensors 18 in einem in der Fig. 6 gezeigten Beobachtungsfenster 201 innerhalb des Unterstützungssystems 10, wobei ein Verschieben des Beobachtungsfensters 201 mit einer Beobachtungsfenstergeschwindigkeit erfolgt, welche unter Verwendung der geschätzten Strömungsgeschwindigkeit bestimmt wird.

Die Einrichtung 50 zum Durchführen einer gepulsten Dopplermessung ist für das Verschieben des Beobachtungsfensters 201 durch Verändern des zeitlichen Abstands zwischen einer Aussendung eines Ultraschall-Impulses und einem Mess-Zeitintervall von Ultraschall-Impuls zu Ultraschall-Impuls ausgelegt.

Darüber hinaus weist das Steuer- und Verarbeitungsgerät 46 eine Einrichtung 52 zum Ermitteln des mindestens einen Strömungsparameters des Fluids unter Verwendung zumindest eines Messergebnisses der gepulsten Dopplermessung oder eines Messergebnisses der gepulsten Dopplermessung und der Beobachtungsfenstergeschwindigkeit auf. Die Einrichtung 52 zum Ermitteln des mindestens einen Strömungsparameters des Fluids ist für das Ermitteln einer Strömungsgeschwindigkeit des Fluids unter Verwendung eines Messergebnisses der gepulsten Dopplermessung und der Beobachtungsfenstergeschwindigkeit durch eine Addition der Beobachtungsfenstergeschwindigkeit mit einer auf Basis der gepulten Dopplermessung ermittelten Relativgeschwindigkeit ausgelegt.

Die Einrichtung 48 zum Schätzen der Strömungsgeschwindigkeit des Fluids 31 dient für das Schätzen der Strömungsgeschwindigkeit des Fluids 31 auf Basis eines Betriebsparameters einer Strömungsmaschine des Unterstützungssystems 10.

Die Beobachtungsfenstergeschwindigkeit des Beobachtungsfensters 201 der Einrichtung zum Durchführen einer gepulsten Dopplermessung ist für das Transformieren einer Dopplerverschiebung in einen mehrdeutigkeitsfrei darstellbaren Bereich ausgelegt, wobei die Beobachtungsfenstergeschwindigkeit im Wesentlichen einer in der Einrichtung 48 zum Schätzen der Strömungsgeschwindigkeit des Fluids 31 geschätzten Strömungsgeschwindigkeit entspricht.

In dem System sind die Beobachtungsfenstergeschwindigkeit und eine Abtastrate des durch das implantierte, vaskuläre Unterstützungssystem 10 strömenden Fluids 31 aneinander angepasst. Die Beobachtungsfenstergeschwindigkeit ist dabei derart bestimmt, dass das Messergebnis der gepulsten Dopplermessung und eine Dopplerverschiebung durch statische Streuer voneinander beabstandet sind.

## Bezugszeichenliste

| | |
|---|---|
| 10 | Unterstützungssystem |
| 12 | Einlaufkäfig |
| 18 | Ultraschallsensor |
| 19 | Bypass |
| 20 | Herz |
| 21 | linker Ventrikel |
| 22 | Aorta |
| 23 | Aortenklappe |
| 31 | Fluid-Volumenstrom/Blutstrom |
| 32 | Fluid-Gesamtvolumenstrom |
| 40 | Doppler-Frequenzspektrum |
| 41 | Hauptfrequenzkomponente |
| 42 | Peak durch Dopplerverschiebung |
| 43 | Peak durch bewegte Streuer |
| 44 | Flussgeschwindigkeit |
| 45 | System |
| 46 | Steuer- und/oder Verarbeitungsgerät |
| 48 | Einrichtung zum Schätzen der Strömungsgeschwindigkeit |
| 50 | Einrichtung zum Durchführen einer gepulsten Dopplermessung |
| 52 | Einrichtung zum Ermitteln eines Strömungsparameters |

200    Kanal
201    Beobachtungsfenster

**Patentansprüche**

1. Verfahren zur Bestimmung mindestens eines Strömungsparameters von durch einen kanülenartigen Abschnitt eines implantierten, vaskulären Unterstützungssystems (10) strömendem Blut (31), umfassend folgende Schritte:

   a) Schätzen der Strömungsgeschwindigkeit des Bluts (31),
   b) Durchführen einer gepulsten Dopplermessung mittels eines Ultraschallsensors (18) des Unterstützungssystems (10) in einem Beobachtungsfenster (201) innerhalb des kanülenartigen Abschnitts des Unterstützungssystems (10), wobei ein Verschieben des Beobachtungsfensters (201) mit einer Beobachtungsfenstergeschwindigkeit erfolgt, welche unter Verwendung der in Schritt a) geschätzten Strömungsgeschwindigkeit bestimmt wird, und
   c) Ermitteln des mindestens einen Strömungsparameters des Bluts unter Verwendung zumindest eines Messergebnisses der gepulsten Dopplermessung oder eines Messergebnisses der gepulsten Dopplermessung und der Beobachtungsfenstergeschwindigkeit.

2. Verfahren nach Anspruch 1, wobei in Schritt c) eine Strömungsgeschwindigkeit des Bluts unter Verwendung eines Messergebnisses der gepulsten Dopplermessung und der Beobachtungsfenstergeschwindigkeit ermittelt wird.

3. Verfahren nach Anspruch 1 oder 2, wobei in Schritt a) die Schätzung auf Basis eines Betriebsparameters einer Strömungsmaschine des Unterstützungssystems (10) erfolgt.

4. Verfahren nach einem der vorhergehenden Ansprüche, wobei die Beobachtungsfenstergeschwindigkeit so bestimmt wird, dass eine Dopplerverschiebung in einen Bereich transformiert wird, der mehrdeutigkeitsfrei darstellbar ist.

5. Verfahren nach einem der vorhergehenden Ansprüche, wobei die Beobachtungsfenstergeschwindigkeit im Wesentlichen der in Schritt a) geschätzten Strömungsgeschwindigkeit entspricht.

6. Verfahren nach einem der vorhergehenden Ansprüche, wobei zum Verschieben des Beobachtungsfensters (201) der zeitliche Abstand zwischen einer Aussendung eines Ultraschall-Impulses und einem Mess-Zeitintervall von Ultraschall-Impuls zu Ultraschall-Impuls verändert wird; und/oder dass die Beobachtungsfenstergeschwindigkeit und eine Abtastrate aneinander angepasst werden; und/oder dass die Beobachtungsfenstergeschwindigkeit derart bestimmt wird, dass das Messergebnis der gepulsten Dopplermessung und eine Dopplerverschiebung durch statische Streuer voneinander beabstandet sind; und/oder dass in Schritt c) eine Strömungsgeschwindigkeit des Bluts durch eine Addition der Beobachtungsfenstergeschwindigkeit mit einer auf Basis der gepulsten Dopplermessung ermittelten Relativgeschwindigkeit ermittelt wird.

7. Implantierbares, vaskuläres Unterstützungssystem (10), eingerichtet zur Durchführung eines Verfahrens nach einem der Ansprüche 1 bis 7.

8. System mit einem implantierbaren, vaskulären Unterstützungssystem (10), das einen kanülenartigen Abschnitt für das Ansaugen von Blut hat, mit einem Steuer- und/oder Verarbeitungsgerät (46) zur Bestimmung mindestens eines Strömungsparameters eines durch das implantierbare, vaskuläre Unterstützungssystem (10) strömenden Fluids (31), wobei das Steuer- und/oder Verarbeitungsgerät (46) und mit

   a) einer Einrichtung (48) zum Schätzen der Strömungsgeschwindigkeit des Bluts (31), die sich in dem Verarbeitungsgerät (46) befindet; und mit einer
   b) Einrichtung (50) zum Durchführen einer gepulsten Dopplermessung mittels eines Ultraschallsensors (18) in einem Beobachtungsfenster (201) innerhalb des kanülenartigen Abschnitts des Unterstützungssystems (10), wobei die Einrichtung (50) so ausgelegt ist, dass ein Verschieben des Beobachtungsfensters (201) mit einer Beobachtungsfenstergeschwindigkeit erfolgt, welche unter Verwendung der in der Einrichtung (48) zum Schätzen der Strömungsgeschwindigkeit des Fluids (31) geschätzten Strömungsgeschwindigkeit bestimmt wird, und
   c) eine Einrichtung (52) zum Ermitteln des mindestens einen Strömungsparameters des Fluids unter Verwendung zumindest eines Messergebnisses der gepulsten Dopplermessung oder eines Messergebnisses der gepulsten Dopplermessung und der Beobachtungsfenstergeschwindigkeit.

9. System nach Anspruch 8, wobei die Einrichtung (52) zum Ermitteln des mindestens einen Strömungsparameters des Fluids für das Ermitteln einer Strömungsgeschwindigkeit des Fluids unter Verwendung eines Messergebnisses der gepulsten Dopplermessung und der Beobachtungsfenstergeschwindigkeit ausgelegt ist.

10. System nach Anspruch 8 oder 9, wobei die Einrichtung (48) zu Schätzen der Strömungsgeschwindigkeit des Fluids (31) für das Schätzen der Strömungsgeschwindigkeit des Fluids (31) auf Basis eines Betriebsparameters einer Strömungsmaschine des Unterstützungssystems (10) ausgelegt ist.

11. System nach einem der Ansprüche 8 bis 10, wobei die Beobachtungsfenstergeschwindigkeit des Beobachtungsfensters (201) der Einrichtung zum Durchführen einer gepulsten Dopplermessung für das Transformieren einer Dopplerverschiebung in einen mehrdeutigkeitsfrei darstellbaren Bereich ausgelegt ist.

12. System nach einem der Ansprüche 8 bis 11, wobei die Beobachtungsfenstergeschwindigkeit im Wesentlichen einer in der Einrichtung zum Schätzen der Strömungsgeschwindigkeit des Fluids (31) geschätzten Strömungsgeschwindigkeit entspricht.

13. System nach einem der Ansprüche 8 bis 12, wobei die Einrichtung (50) zum Durchführen einer gepulsten Dopplermessung für das Verschieben des Beobachtungsfensters (201) durch Verändern des zeitlichen Abstands zwischen einer Aussendung eines Ultraschall-Impulses und einem Mess-Zeitintervall von Ultraschall-Impuls zu Ultraschall-Impuls ausgelegt ist.

14. System nach einem der Ansprüche 8 bis 13, wobei die Beobachtungsfenstergeschwindigkeit und eine Abtastrate des durch das implantierte, vaskuläre Unterstützungssystem (10) strömenden Fluids (31) aneinander angepasst sind.

15. System nach einem der Ansprüche 8 bis 14, wobei die Beobachtungsfenstergeschwindigkeit derart bestimmt ist, dass das Messergebnis der gepulsten Dopplermessung und eine Dopplerverschiebung durch statische Streuer voneinander beabstandet sind; und/oderdass die Einrichtung (52) zum Ermitteln des mindestens einen Strömungsparameters des Fluids für das Ermitteln der Strömungsgeschwindigkeit des Fluids durch eine Addition der Beobachtungsfenstergeschwindigkeit mit einer auf Basis der gepulten Dopplermessung ermittelten Relativgeschwindigkeit ausgelegt ist.

**Claims**

1. Method for determining at least one flow parameter of blood (31) flowing through a cannula-like portion of an implanted vascular assist system (10), comprising the following steps:

a) estimating the flow velocity of the blood (31),
b) performing a pulsed Doppler measurement by means of an ultrasonic sensor (18) of the assist system (10) in an observation window (201) within the cannula-like portion of the assist system (10), wherein the observation window (201) is displaced at an observation window velocity which is determined using the flow velocity estimated in step a), and
c) identifying the at least one flow parameter of the blood using at least one measurement result of the pulsed Doppler measurement or a measurement result of the pulsed Doppler measurement and the observation window velocity.

2. Method according to claim 1, wherein in step c) a flow velocity of the blood is identified using a measurement result of the pulsed Doppler measurement and the observation window velocity.

3. Method according to either claim 1 or claim 2, wherein in step a) the estimation is made on the basis of an operating parameter of a fluid-flow machine of the assist system (10).

4. Method according to any of the preceding claims, wherein the observation window velocity is determined such that a Doppler shift is transformed into a range that can be displayed without ambiguity.

5. Method according to any of the preceding claims, wherein the observation window velocity substantially corresponds to the flow velocity estimated in step a).

**6.** Method according to any of the preceding claims, wherein, in order to displace the observation window (201), the time interval between an ultrasonic pulse being emitted and a measurement time interval from ultrasonic pulse to ultrasonic pulse is changed; and/or in that the observation window velocity and a sampling rate are adapted to one another; and/or in that the observation window velocity is determined such that the measurement result of the pulsed Doppler measurement and a Doppler shift caused by static scattering are spaced apart from one another; and/or in that, in step c), a flow velocity of the blood is identified by adding the observation window velocity and a relative speed identified on the basis of the pulsed Doppler measurement.

**7.** Implantable vascular assist system (10), which is configured to carry out a method according to any of claims 1 to 7.

**8.** System comprising an implantable vascular assist system (10) which has a cannula-like portion for sucking in blood, comprising a control and/or processing device (46) for determining at least one flow parameter of a fluid (31) flowing through the implantable vascular assist system (10), wherein the control and/or processing device (46) and comprising

a) a device (48) for estimating the flow velocity of the blood (31) located in the processing device (46); and comprising

b) a device (50) for performing a pulsed Doppler measurement by means of an ultrasonic sensor (18) in an observation window (201) within the cannula-like portion of the assist system (10), wherein the device (50) is designed such that the observation window (201) is displaced at an observation window velocity which is determined using the flow velocity estimated in the device (48) for estimating the flow velocity of the fluid (31), and

c) a device (52) for identifying the at least one flow parameter of the fluid using at least one measurement result of the pulsed Doppler measurement or a measurement result of the pulsed Doppler measurement and the observation window velocity.

**9.** System according to claim 8, wherein the device (52) for identifying the at least one flow parameter of the fluid is designed to identify a flow velocity of the fluid using a measurement result of the pulsed Doppler measurement and the observation window velocity.

**10.** System according to either claim 8 or claim 9, wherein the device (48) for estimating the flow velocity of the fluid (31) is designed to estimate the flow velocity of the fluid (31) on the basis of an operating parameter of a fluid-flow machine of the assist system (10).

**11.** System according to any of claims 8 to 10, wherein the observation window velocity of the observation window (201) of the device is designed to perform a pulsed Doppler measurement for transforming a Doppler shift into a range that can be displayed without ambiguity.

**12.** System according to any of claims 8 to 11, wherein the observation window velocity substantially corresponds to a flow velocity estimated in the device for estimating the flow velocity of the fluid (31).

**13.** System according to any of claims 8 to 12, wherein the device (50) for performing a pulsed Doppler measurement is designed to displace the observation window (201) by changing the time interval between an ultrasonic pulse being emitted and a measurement time interval from ultrasonic pulse to ultrasonic pulse.

**14.** System according to any of claims 8 to 13, wherein the observation window velocity and a sampling rate of the fluid (31) flowing through the implanted vascular assist system (10) are adapted to one another.

**15.** System according to any of claims 8 to 14, wherein the observation window velocity is determined such that the measurement result of the pulsed Doppler measurement and a Doppler shift caused by static scattering are spaced apart from one another; and/or in that the device (52) for identifying the at least one flow parameter of the fluid is designed to identify the flow velocity of the fluid by adding the observation window velocity and a relative speed identified on the basis of the pulsed Doppler measurement.

**Revendications**

**1.** Procédé permettant de déterminer au moins un paramètre d'écoulement du sang (31) s'écoulant à travers une section en forme de canule d'un système d'assistance (10) vasculaire implanté, comprenant les étapes suivantes :

a) estimation de la vitesse d'écoulement du sang (31),

b) réalisation d'une mesure Doppler pulsée au moyen d'un capteur à ultrasons (18) du système d'assistance (10) dans une fenêtre d'observation (201) à l'intérieur de la section en forme de canule du système d'assistance (10), dans lequel un déplacement de la fenêtre d'observation (201) est effectué à une vitesse de fenêtre d'observation qui est déterminée à l'aide de la vitesse d'écoulement estimée à l'étape a), et

c) définition de l'au moins un paramètre d'écoulement du sang à l'aide d'au moins un résultat de mesure de la mesure Doppler pulsée ou d'un résultat de mesure de la mesure Doppler pulsée et de la vitesse de fenêtre d'observation.

2. Procédé selon la revendication 1, dans lequel, à l'étape c), une vitesse d'écoulement du sang est définie à l'aide d'un résultat de mesure de la mesure Doppler pulsée et de la vitesse de fenêtre d'observation.

3. Procédé selon la revendication 1 ou 2, dans lequel, à l'étape a), l'estimation est effectuée sur la base d'un paramètre de fonctionnement d'une turbomachine du système d'assistance (10).

4. Procédé selon l'une des revendications précédentes, dans lequel la vitesse de fenêtre d'observation est déterminée de sorte qu'un décalage Doppler est transformé en une zone qui peut être représentée sans ambiguïté.

5. Procédé selon l'une des revendications précédentes, dans lequel la vitesse de fenêtre d'observation correspond sensiblement à la vitesse d'écoulement estimée à l'étape a).

6. Procédé selon l'une des revendications précédentes, dans lequel, pour le déplacement de la fenêtre d'observation (201), l'écart temporel entre une émission d'une impulsion ultrasonore et un intervalle de temps de mesure entre une impulsion ultrasonore et une autre impulsion ultrasonore est modifié ; et/ou en ce que la vitesse de fenêtre d'observation et une fréquence d'échantillonnage sont adaptées l'une à l'autre ; et/ou en ce que la vitesse de fenêtre d'observation est déterminée de telle sorte que le résultat de mesure de la mesure Doppler pulsée et un décalage Doppler sont espacés l'un de l'autre par des diffuseurs statiques ; et/ou en ce que, à l'étape c), une vitesse d'écoulement du sang est définie par une addition de la vitesse de fenêtre d'observation à une vitesse relative définie sur la base de la mesure Doppler pulsée.

7. Système d'assistance (10) vasculaire implantable, configuré pour la réalisation d'un procédé selon l'une des revendications 1 à 7.

8. Système comportant un système d'assistance (10) vasculaire implantable qui possède une section en forme de canule destinée à aspirer le sang, comportant un appareil de commande et/ou de traitement (46) pour la détermination d'au moins un paramètre d'écoulement d'un fluide (31) s'écoulant à travers le système d'assistance (10) vasculaire implantable, dans lequel l'appareil de commande et/ou de traitement (46) et comportant

a) un dispositif (48) permettant d'estimer la vitesse d'écoulement du sang (31), lequel dispositif se trouve dans l'appareil de traitement (46) ; et comportant un

b) dispositif (50) permettant de réaliser une mesure Doppler pulsée au moyen d'un capteur à ultrasons (18) dans une fenêtre d'observation (201) à l'intérieur de la section en forme de canule du système d'assistance (10), dans lequel le dispositif (50) est conçu de sorte qu'un déplacement de la fenêtre d'observation (201) à une vitesse de fenêtre d'observation est effectué, laquelle vitesse est déterminée à l'aide de la vitesse d'écoulement estimée dans le dispositif (48) permettant d'estimer la vitesse d'écoulement du fluide (31), et

c) un dispositif (52) permettant de définir l'au moins un paramètre d'écoulement du fluide à l'aide d'au moins un résultat de mesure de la mesure Doppler pulsée ou d'un résultat de mesure de la mesure Doppler pulsée et de la vitesse de fenêtre d'observation.

9. Système selon la revendication 8, dans lequel le dispositif (52) permettant de définir l'au moins un paramètre d'écoulement du fluide est configuré pour définir une vitesse d'écoulement du fluide à l'aide d'un résultat de mesure de la mesure Doppler pulsée et de la vitesse de fenêtre d'observation.

10. Système selon la revendication 8 ou 9, dans lequel le dispositif (48) permettant d'estimer la vitesse d'écoulement du fluide (31) est configuré pour estimer la vitesse d'écoulement du fluide (31) sur la base d'un paramètre de fonctionnement d'une turbomachine du système d'assistance (10).

11. Système selon l'une des revendications 8 à 10, dans lequel la vitesse de fenêtre d'observation de la fenêtre d'observation (201) du dispositif permettant de réaliser une mesure Doppler pulsée est configurée pour transformer un décalage Doppler en une zone pouvant être représentée sans ambiguïté.

12. Système selon l'une des revendications 8 à 11, dans lequel la vitesse de fenêtre d'observation correspond sensiblement à une vitesse d'écoulement estimée dans le dispositif permettant d'estimer la vitesse d'écoulement du fluide (31).

13. Système selon l'une des revendications 8 à 12, dans lequel le dispositif (50) permettant de réaliser une mesure Doppler pulsée est configuré pour déplacer la fenêtre d'observation (201) en modifiant l'écart temporel entre une émission d'une impulsion ultrasonore et un intervalle de temps de mesure entre une impulsion ultrasonore et une autre impulsion ultrasonore.

14. Système selon l'une des revendications 8 à 13, dans lequel la vitesse de fenêtre d'observation et une fréquence d'échantillonnage du fluide (31) s'écoulant à travers le système d'assistance (10) vasculaire implanté sont adaptées l'une à l'autre.

15. Système selon l'une des revendications 8 à 14, dans lequel la vitesse de fenêtre d'observation est déterminée de telle sorte que le résultat de mesure de la mesure Doppler pulsée et un décalage Doppler sont espacés l'un de l'autre par des diffuseurs statiques ; et/ou en ce que le dispositif (52) permettant de définir l'au moins un paramètre d'écoulement du fluide est configuré pour définir la vitesse d'écoulement du fluide par une addition de la vitesse de fenêtre d'observation à une vitesse relative définie sur la base de la mesure Doppler pulsée.

# Fig. 1

# Fig. 2

# Fig. 3

a)           b)           c)

110           120           130

# Fig. 4

$A$ [a.u.]

41

40

$f$ [Hz]

-20000    -10000    0    10000    20000

## Fig. 5

## Fig. 6

## Fig. 7a

## Fig. 7b

# Fig. 8a

# Fig. 8b

# Fig. 8c

# Fig. 9a

# Fig. 9b

Fig. 10

45

46

48

50

52

31

10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2008133006 A1
- US 20160000983 A1
- DE 19520920 A1